# EUROPEAN PATENT APPLICATION

(11) **EP 2 444 810 A2**
(43) Date of publication of application: **25.04.2012**
(21) Application number: 11179703.1
(22) Date of filing: 02.10.2003
(51) Int. Cl.: G01N 33/573, C12N 9/64, C12Q 1/37

(54) **Proteases mutants with altered specificity and uses thereof**

(30) Priority: 02.10.2002 US 415388 P
(62) Divisional of application: 03774608.8
(71) Applicant: Catalyst Biosciences, Inc., South San Francisco, CA 94080 (US); The Regents Of The University Of California, Oakland, CA 94607 (US)
(72) Inventor: Nguyen, Jack, San Francisco, CA California 94110 (US); Thanos, Chris, San Francisco, CA California 94121 (US); Ruggles, Sandra Waugh, Sunnyvale, CA California 94086 (US); Craik, Charles S, San Francisco, CA California 94131 (US)
(74) Representative: Baldock, Sharon Claire

(57) **Abstract**

Methods for generating proteases with altered specificity for the target molecules they cleave is disclosed. The invention further discloses methods of using these proteases to treat diseases in which the target proteins are involved with. Cleaving certain target proteins at certain substrate sequences with a protease is a method for treating these pathologies.

## Description

### BACKGROUND OF THE INVENTION

Enzymes are used within a wide range of applications. An important group of enzymes is the proteases, which cleave proteins. Many proteases cleave target proteins specifically at defined substrate sequences. This tendency for specific cleavage by proteases is referred to as substrate, or substrate sequence, specificity. Substrate specificities associated with different members of the diverse families of proteolytic enzymes can be attributed, in part, to different sets of amino acids within the binding domain, that are utilized by each enzyme family for substrate recognition and catalysis. A rational approach to engineering mutant enzymes has been successful for several proteases. A conserved amino acid residue (glycine 166), known from crystallographic data to reside within the binding cleft, of subtilisin was changed to one of several different amino acid residues. The resulting enzyme derivatives showed dramatic changes in specificity toward substrates with increasing hydrophobicity and amino acid size (Wells, et al., Cold Spring Harb. Symp. Quant. Biol., (1987) 52: 647-52.). Another bacterially encoded serine endopeptidase, α-lytic protease, has also been rationally altered by changing methionine 192 to an alanine. The resulting mutation within the active site of the enzyme appears to have increased structural flexibility of the enzyme active site. The resulting α-lytic protease derivative has a broader substrate specificity towards larger, more hydrophobic targets (Bone, et al., Biochemistry, 1991, (43) :10388-98).

The serine proteases are an extensively studied family of related endopeptidases, characterized by their so-called catalytic triad: Asp His Ser. Within a family of similar proteins, the regions of conserved primary, secondary and tertiary structure tend to include the residues involved in the active site(s), as well as other residues important to activity. For example, the members of the catalytic triad are far apart in the primary structures (amino acid sequence) of serine proteases, but these residues are brought to within bond forming distance by the tertiary structure (or folding), of these proteins.

Serine proteases differ markedly in substrate sequence recognition properties: some are highly specific (*i.e.*, the proteases involved in blood coagulation and the immune complement system); some are only partially specific (*i.e.*, the mammalian digestive proteases trypsin and chymotrypsin); and others, like subtilisin, a bacterial protease, are completely non-specific. Despite these differences in specificity, the catalytic mechanism of serine proteases is well conserved, consisting of a substrate sequence-binding site that correctly positions the scissile peptide in the active site with five hydrogen-bonds. Once the peptide is bound, a hydrogen-bond network between the three invariant residues of the catalytic triad catalyzes the hydrolysis of the peptide bond. This large family of proteases can be broadly divergent in their sequence specificities despite being highly conserved in their mechanism of catalysis.

### SUMMARY OF THE INVENTION

The present invention is drawn to the generation and screening of proteases that cleave proteins known to be involved in disease. The resultant proteins may be used as agents for *in vivo* therapy.

The invention is broadly drawn to the modification of proteases to alter their substrate sequence specificity, so that the modified proteases cleave a target protein which is involved with or causes a pathology. In one embodiment of the invention, this modified protease is a serine protease. In another embodiment of the invention, this modified protease is a cysteine protease.

One embodiment of the invention involves generating a library of protease sequences to be used to screen for modified proteases that cleave a desired target protein at a desired substrate sequence. In one aspect of this embodiment, each member of the library is a protease scaffold with a number of mutations made to each member of the library. A protease scaffold has the same or a similar sequence to a known protease. In one embodiment, this scaffold is a serine protease. In another embodiment of the invention, this scaffold is a cysteine protease. The cleavage activity of each member of the library is measured using the desired substrate sequence from the desired target protein. As a result, proteases with the highest cleavage activity with regard to the desired substrate sequence are detected.

In another aspect of this embodiment, the number of mutations made to the protease scaffold is 1, 2-5 (*e.g.* 2, 3, 4 or 5), 5-10 (e.g. 5, 6, 7, 8, 9 or 10), or 10-20 (e.g. 10, 11, 12, 13, 14, 15, 16, 17, 18, 19 or 20).

In another aspect of this embodiment, the known protease scaffold can include the amino acid sequence of trypsin, chymotrypsin, substilisin, thrombin, plasmin, Factor Xa, urokinase type plasminogen activator (uPA), tissue plasminogen activator (tPA), membrane type serine protease-1 (MTSP-1), granzyme A, granzyme B, granzyme M, elastase, chymase, papain, neutrophil elastase, plasma kallikrein, urokinase type plasminogen activator, complement factor serine proteases, ADAMTS13, neural endopeptidase/neprilysin, furin, or cruzain.

In another aspect of this embodiment, the target protein is involved with a pathology, for example, the target protein causes the pathology. The pathology can be e.g., rheumatoid arthritis, sepsis, cancer, acquired immunodeficiency syndrome, respiratory tract infections, influenza, cardiovascular disease, or asthma.

In another aspect of this embodiment, the activity of the detected protease is increased by at least 10-fold, 100-fold, or 1000-fold over the average activity of the library.

Another embodiment of the invention, involves generating a library of substrate sequences to be used to screen a modified protease in order to detect which substrate sequence(s) the modified protease cleaves most efficiently. The members of the library are made up of randomized amino acid sequences, and the cleavage activity of each member of the library by the protease is measured. Substrate sequences which are cleaved most efficiently by the protease are detected.

In another aspect of this embodiment, the substrate sequence in a library is 4, 5, 6, 7, 8, 9, 10, 11, 11, 12, 13, 14, 15, 16, 17, 18, 19 or 20 amino acids long.

In another aspect of this embodiment, the substrate sequence is a part of a target protein. This target protein can be involved in a pathology. For example, the target protein is one which a causes a pathology. In another aspect of this embodiment, this pathology is rheumatoid arthritis, sepsis, cancer, acquired immunodeficiency syndrome, respiratory tract infections, influenza, cardiovascular disease, or asthma.

In another aspect of this embodiment, the efficiency of cleavage of the detected substrate sequence is increased by at least 10-fold, at least 100-fold, or at least 1000-fold over the average activity of the library.

In yet another embodiment, the invention includes a method for treating a patient having a pathology. The method involves administering to the patient a protease that cleaves a target protein involved with the pathology, so that cleaving the protein treats the pathology.

In one aspect of this embodiment, the pathology can be rheumatoid arthritis, sepsis, cancer, acquired immunodeficiency syndrome, respiratory tract infections, influenza, cardiovascular disease, or asthma. In another aspect of this embodiment, the protease can be a serine protease. In another embodiment of the invention, this modified protease is a cysteine protease. In another aspect of this embodiment, the target protein causes the pathology.

The patient having a pathology, e.g. the patient treated by the methods of this invention can be a mammal, or more particularly, a human.

In another aspect of the embodiment the target protein can be tumor necrosis factor, tumor necrosis factor receptor, interleukin-1, interleukin-1 receptor, interleukin-2, interleukin-2 receptor, interleukin-4, interleukin-4 receptor, interleukin-5, interleukin-5 receptor, interleukin-12, interleukin-12 receptor, interleukin-13, interleukin-13 receptor, p-selectin, p-selectin glycoprotein ligand, Substance P, the Bradykinins, PSGL, factor IX, immunoglobulin E, immunoglobulin E receptor, CCR5, CXCR4, glycoprotein 120, glycoprotein 41, CD4, hemaglutinin, respiratory syncytium virus fusion protein, B7, CD28, CD2, CD3, CD4, CD40, vascular endothelial growth factor, VEGF receptor, fibroblast growth factor, endothelial growth factor, EGF receptor, TGF receptor, transforming growth factor, Her2, CCR1, CXCR3, CCR2, Src, Akt, Bc1-2, BCR-Abl, glucagon synthase kinase-3, cyclin dependent kinase-2 (cdk-2), or cyclin dependent kinase-4 (cdk-4).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and are not intended to be limiting.

Other features and advantages of the invention will be apparent from the following detailed description and claims.

### BRIEF DESCRIPTION OF THE DRAWINGS

Figure 1 is a schematic of the amino acid sequence of caspase-3, and a table containing the amino acid sequence of caspase 3 (SEQ ID NO:1).
Figure 2 is a diagram from X-ray crystallography showing the structure of caspase-3 focusing on the inactivation sequence cleaved by I99A/N218A granzyme B, along with a diagram showing the amino acid sequence of the inactivation sequence site of the enzyme (SEQ ID NO:2),
Figure 3A shows a series of bar graphs showing the substrate specificity of wild-type granzyme B versus the substrate specificity of I99A/N219A granzyme B at P2, P3 and P4. Figure 3B shows a table containing kinetic data derived from the graphs in Figure 3A.
Figure 4 shows a series of graphs from MALDI mass spectrometry of a peptide corresponding to the inactivation sequence in caspase-3 in the presence of wild-type and I99A/N219A granzyme B.
Figure 5 depicts a SDS PAGE gel showing bands for cleavage products of caspase-3 small subunit by wild-type and I99A/N219A granzyme B.
Figure 6A shows a graph plotting caspase-3 activity over time in the presence of wild-type granzyme B and I99A/N219A granzyme B. Figure 6B shows a bar graph showing the Vmax of the activity of caspase-3 in the presence of wild-type and I99A/N219A granzyme B.
Figure 7A shows a bar graph plotting apoptosis in cell lysates as measured by caspase-3 activity in the presence of I99A/N218A granzyme B. Figure 7B shows a bar graph plotting apoptosis in cell lysates as measured by caspase-3 activity in the presence of wildtype and increasing concentrations of I99A/N218A granzyme B.

### DETAILED DESCRIPTION OF THE INVENTION

Prior to setting forth the invention in detail, certain terms used herein will be defined.

The term "allelic variant" denotes any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term "allelic variant" is also used herein to denote a protein encoded by an allelic variant of a gene.

The term "complements of polynucleotide molecules" denotes polynucleotide molecules having a complementary base sequence and reverse orientation as compared to a reference sequence. For example, the sequence 5' ATGCACGGG 3' is complementary to 5' CCCGTGCAT 3'.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (*i.e.*, GAU and GAC triplets each encode Asp).

A "DNA construct" is a single or double stranded, linear or circular DNA molecule that comprises segments of DNA combined and juxtaposed in a manner not found in nature. DNA constructs exist as a result of human manipulation, and include clones and other copies of manipulated molecules.

A "DNA segment" is a portion of a larger DNA molecule having specified attributes. For example, a DNA segment encoding a specified polypeptide is a portion of a longer DNA molecule, such as a plasmid or plasmid fragment, that, when read from the 5' to the 3' direction, encodes the sequence of amino acids of the specified polypeptide.

The term "expression vector" denotes a DNA construct that comprises a segment encoding a polypeptide of interest operably linked to additional segments that provide for its transcription in a host cell. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both.

The term "isolated", when applied to a polynucleotide molecule, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones, as well as synthetic polynucleotides. Isolated DNA molecules of the present invention may include naturally occurring 5' and 3' untranslated regions such as promoters and terminators. The identification of associated regions will be evident to one of ordinary skill in the art (see for example, Dynan and Tijan, Nature 316:774-78, 1985). When applied to a protein, the term "isolated" indicates that the protein is found in a condition other than its native environment, such as apart from blood and animal tissue. In a preferred form, the isolated protein is substantially free of other proteins, particularly other proteins of animal origin. It is preferred to provide the protein in a highly purified form, *i.e.*, at least 90% pure, preferably greater than 95% pure, more preferably greater than 99% pure.

The term "operably linked", when referring to DNA segments, denotes that the segments are arranged so that they function in concert for their intended purposes, *e.g*. transcription initiates in the promoter and proceeds through the coding segment to the terminator.

The term "ortholog" denotes a polypeptide or protein obtained from one species that is the functional counterpart of a polypeptide or protein from a different species. Sequence differences among orthologs are the result of speciation.

The term "polynucleotide" denotes a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules. The length of a polynucleotide molecule is given herein in terms of nucleotides (abbreviated "nt") or base pairs (abbreviated "bp"). The term "nucleotides" is used for both single- and double-stranded molecules where the context permits. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired. Such unpaired ends will, in general, not exceed 20 nt in length.

The term "promoter" denotes a portion of a gene containing DNA sequences that provide for the binding of RNA polymerase and initiation of transcription. Promoter sequences are commonly, but not always, found in the 5' non-coding regions of genes.

A "protease" is an enzyme that cleaves peptide bonds in proteins. A "protease precursor" is a relatively inactive form of the enzyme that commonly becomes activated upon cleavage by another protease.

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

The term "substrate sequence" denotes a sequence that is specifically targeted for cleavage by a protease.

The term "target protein" denotes a protein that is specifically cleaved at its substrate sequence by a protease.

The terms "S1-S4" refer to the residues in a protease that make up the substrate sequence binding pocket. They are numbered sequentially from the recognition site N-terminal to the site of proteolysis- the scissile bond.

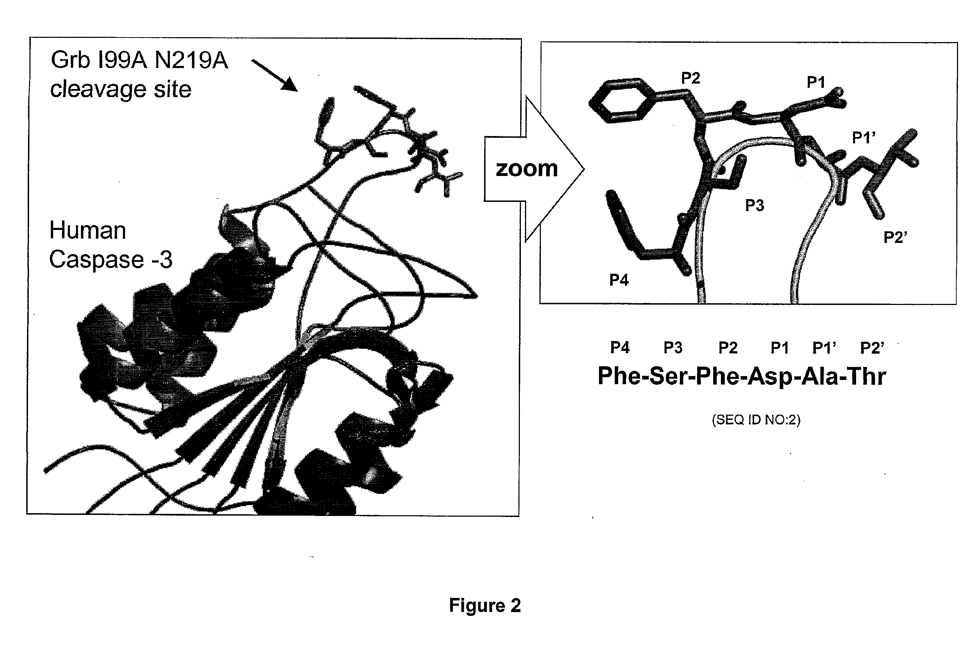

The terms "P1-P4" and "P1'-P4'"' refer to the residues in a peptide to be cleaved that specifically interact with the S1-S4 residues found above. P1-P4 generally comprise the substrate sequence. P1-P4 are the positions on the N-terminal side of the cleavage site, whereas P1'-P4' are the positions to the C-terminal side of the cleavage site. (*See* Figure 2).

The term "scaffold" refer to an existing protease to which various mutations are made. Generally, these mutations change the specificity and activity of the scaffold.

An "isolated" or "purified" polypeptide or protein or biologically-active portion thereof is substantially free of cellular material or other contaminating proteins from the cell or tissue source from which the protease protein is derived, or substantially free from chemical precursors or other chemicals when chemically synthesized. The language "substantially free of cellular material" includes preparations of protease proteins in which the protein is separated from cellular components of the cells from which it is isolated or recombinantly-produced. In one embodiment, the language "substantially free of cellular material" includes preparations of protease proteins having less than about 30% (by dry weight) of non-protease proteins (also referred to herein as a "contaminating protein"), more preferably less than about 20% of non-protease proteins, still more preferably less than about 10% of non-protease proteins, and most preferably less than about 5% of non-protease proteins. When the protease protein or biologically-active portion thereof is recombinantly-produced, it is also preferably substantially free of culture medium, *i.e.*, culture medium represents less than about 20%, more preferably less than about 10%, and most preferably less than about 5% of the volume of the protease protein preparation.

The language "substantially free of chemical precursors or other chemicals" includes preparations of protease proteins in which the protein is separated from chemical precursors or other chemicals that are involved in the synthesis of the protein. In one embodiment, the language "substantially free of chemical precursors or other chemicals" includes preparations of protease proteins having less than about 30% (by dry weight) of chemical precursors or non-protease chemicals, more preferably less than about 20% chemical precursors or non-protease chemicals, still more preferably less than about 10% chemical precursors or non-protease chemicals, and most preferably less than about 5% chemical precursors or non-protease chemicals.

The present invention is drawn to methods for generating and screening proteases to cleave target proteins at a given substrate sequence. Proteases are protein-degrading enzymes that recognize an amino acid or an amino acid substrate sequence within a target protein. Upon recognition of the substrate sequence, proteases catalyze the hydrolysis or cleavage of a peptide bond within a target protein. Such hydrolysis of the target protein may inactivate it, depending on the location of peptide bond within the context of the full-length sequence of the target sequence. The specificity of proteases can be altered through protein engineering. If a protease is engineered to recognize a substrate sequence within a target protein or proteins that would *(i.)* alter the function *i*.*e*. by inactivation of the target protein(s) upon catalysis of peptide bond hydrolysis and, *(ii.)* the target protein(s) are recognized or unrecognized as points of molecular intervention for a particular disease or diseases, then the engineered protease has a therapeutic effect via a proteolysis-mediated inactivation event. In particular, proteases can be engineered to cleave receptors between their transmembrane and cytokine binding domains. The stalk regions which function to tether protein receptors to the surface of a cell or loop regions are thereby disconnected from the globular domains in a polypeptide chain.

In one embodiment, the target protein to be cleaved is involved with a pathology, where cleaving the target protein at a given substrate sequence serves as a treatment for the pathology.

In one embodiment, the protease cleaves a protein involved with rheumatoid arthritis. For example, the protease cleaves the TNF receptor between the transmembrane domain and the cytokine binding domain. This cleavage can inactivate the receptor. Rheumatoid arthritis is thereby treated by inhibiting the action of tumor necrosis factor (TNF).

The protease cleaves the same targets as activated protein C. This cleavage can attenuate the blood coagulation cascade. Sepsis is thereby treated by supplementing the action of protein C.

The protease cleaves cell surface molecules that are responsible for tumorigenicity, preventing the spread of cancer. For example, cleavage of cell surface molecules can inactivate their ability to transmit extracellular signals, especially cell proliferation signals. Without these signals, cancer cells often cannot proliferate. The protease of the invention could therefore, be used to treat cancer. In another aspect of this embodiment, the protease could cleave any target protein that is responsible for the spread of cancer. Cleaving a target protein involved in cell cycle progression could inactivate the ability of the protein to allow the cell cycle to go forward. Without the progression of the cell cycle, cancer cells could not proliferate. Therefore, the proteases of the invention could be used to treat cancer.

In another embodiment of the invention, the protease cleaves membrane fusion proteins found on human immunodeficiency virus (HIV), Respiratory Syncytial Virus (RSV), or influenza, inhibiting these virus' ability to infect cells. Without these membrane proteins, these viruses would not be able to infect cells. Therefore, the protease could be used to treat or prevent infection by HIV, RSVm or influenza.

In another embodiment of the invention, the protease cleaves the same target protein as plasminogen activator. By cleaving the target of plasminogen activator, the thrombolytic cascade is activated. In the case of a stroke or heart attack caused by a blood clot, the protease can be used as a treatment for cardiovascular disease.

In another embodiment of the invention, the protease cleaves cytokines or receptors that are involved in inflammation as a treatment for asthma or other pathologies associated with inflammation. By cleaving the cytokine or receptors, the protease can inactivate the signaling cascade involved with many inflammatory processes. The protease can thereby be used to treat inflammation and related pathologies.

In another embodiment of the invention, the protease cleaves signaling molecules that are involved in various signal cascades, including the signaling cascade responsible for the regulation of apoptosis. For example, the protease cleaves a caspase. This caspase can be, for example, caspase-3. By cleaving a protein involved in a signal cascade, the protease can be used to inactivate or modulate the signal cascade.

In some examples, the engineered protease is designed to cleave any of the target proteins in Table 1, thereby inactivating the activity of the protein. The protease can be used to treat a pathology associated with that protein, by inactivating one of the target proteins.

**Table 1**

| **Target** | **Indication** | **Molecule class** |
|---|---|---|
| IL-5/IL-5R | Asthma | Cytokine |
| IL-1/IL-1R | Asthma, inflammation, Rheumatic disorders | Cytokine |
| IL-13/IL-13R | Asthma | Cytokine |
| IL-12/IL-12R | Immunological disorders | Cytokine |
| IL-4/IL-4R | Asthma | Cytokine |
| TNF/TNFR | Asthma, Crohn's disease, | Cytokine |
| | HIV infection, inflammation, psoriasis, rheumatoid arthritis | |
| CCR5/CXCR4 | HIV infection | GPCR |
| gp120/gp41 | HIV infection | Fusion protein |
| CD4 | HIV infection | Receptor |
| Hemaglutinin | Influenza infection | Fusion protein |
| RSV fusion protein | RSV infection | Fusion protein |
| B7/CD28 | Graft-v.-host disorder, rheumatoid arthritis, transplant rejection, diabetes mellitus | Receptor |
| IgE/IgER | Graft-v.-host disorder, transplant rejection | Receptor |
| CD2,CD3,CD4,CD40 | Graft-v.-host disorder, transplant rejection, psoriasis, | Receptor |
| IL-2/IL-2R | Autoimmune disorders, graft-v.-host disorder, rheumatoid arthritis | Cytokine |
| VEGF,FGF,EGF,TGF | Cancer | Cytokine |
| Her2/neu | Cancer | Receptor |
| CCR1 | Multiple sclerosis | GPCR |
| CXCR3 | Multiple sclerosis, rheumatoid arthritis | GPCR |
| CCR2 | Atherosclerosis, rheumatoid arthritis | GPCR |
| Src | Cancer, osteoporosis | Kinase |
| Akt | Cancer | Kinase |
| Bcl-2 | Cancer | Protein-protein |
| BCR-Abl | Cancer | Kinase |
| GSK-3 | Diabetes | Kinase |
| cdk-2/cdk-4 | Cancer | Kinase |

The protease scaffolds are also any of the proteins disclosed below in Table 2.

**Table 2**

| **Code** | **Name** | **Gene** | **Link** | **Locus** |
|---|---|---|---|---|
| S01.010 | granzyme B, human-type | GZMB | 3002 | 14q11.2 |
| S01.011 | testisin | PRSS21 | 10942 | 16p13.3 |
| S01.015 | tryptase beta 1 (Homo sapiens) (III) | TPSB1 | 7177 | 16p13.3 |
| S01.017 | kallikrein hK5 | KLK5 | 25818 | 19q13.3-q13.4 |
| S01.019 | corin | | 10699 | 4p13-p12 |
| S01.020 | kallikrein 12 | KLK12 | 43849 | 19q13.3-q13.4 |
| S01.021 | DESC1 protease | | 28983 | 4q13.3 |
| S01.028 | tryptase gamma 1 | TPSG1 | 25823 | 16p13.3 |
| S01.029 | kallikrein hK14 | KLK14 | 43847 | 19q13.3-q13.4 |
| S01.033 | hyaluronan-binding serine protease (HGF activator-like protein) | HABP2 | 3026 | 10q25.3 |
| S01.034 | transmembrane protease, serine 4 | TMPRSS4 | 56649 | 11q23.3 |
| S01.054 | tryptase delta 1 (Homo sapiens) | TPSD1 | 23430 | 16p13.3 |
| S01.074 | marapsin | | 83886 | 16p13.3 |
| S01.075 | tryptase homologue 2 (Homo sapiens) | | 260429 | |
| S01.076 | tryptase homologue 3 (Homo sapiens) | | | |
| S01.077 | tryptase chromosome 21 (Homo sapiens) | | | 21q |
| S01.079 | transmembrane protease, serine 3 | TMPRSS3 | 64699 | 21q22.3 |
| S01.081 | kallikrein hK15 (Homo sapiens) | | 55554 | 19q13.41 |
| S01.085 | Mername-AA031 peptidase (deduced from ESTs by MEROPS) | | | |
| S01.087 | membrane-type mosaic serine protease | | 84000 | 11q23 |
| S01.088 | Mername-AA038 peptidase | | | |
| S01.098 | Mername-AA128 peptidase (deduced from ESTs by MEROPS) | | | |
| S01.127 | cationic trypsin (Homo sapiens-type) (1 (cationic)) | PRSS1 | 5644 | 7q35 |
| S01.131 | neutrophil elastase | ELA2 | 1991 | 19p13.3 |
| S01.132 | mannan-binding lectin-associated serine protease-3 | | | |
| S01.133 | cathepsin G | CTSG | 1511 | 14q11.2 |
| S01.134 | myeloblastin (proteinase 3) | PRTN3 | 5657 | 19p13.3 |
| S01.135 | granzyme A | GZMA | 3001 | 5q11-q12 |
| S01.139 | granzyme M | GZMM | 3004 | 19p13.3 |
| S01.140 | chymase (human-type) | CMA1 | 1215 | 14q11.2 |
| S01.143 | tryptase alpha (1) | TPS1 | 7176 | 16p13.3 |
| S01.146 | granzyme K | GZMK | 3003 | 5q11-q12 |
| S01.147 | granzyme H | GZMH | 2999 | 14q11.2 |
| S01.152 | chymotrypsin B | CTRB1 | 1504 | 16q23.2-q23.3 |
| S01.153 | pancreatic elastase | ELA1 | 1990 | 12q13 |
| S01.154 | pancreatic endopeptidase E (A) | | 10136 | 1p36.12 |
| S01.155 | pancreatic elastase II (IIA) | | 63036 | 12 |
| S01.156 | enteropeptidase | PRSS7 | 5651 | 21q21 |
| S01.157 | chymotrypsin C | | 11330 | 1 |
| S01.159 | prostasin | PRSS8 | 5652 | 16p11.2 |
| S01.160 | kallikrein 1 | KLK1 | 3816 | 19q13.2-q13.4 |
| S01.161 | kallikrein hK2 (Homo sapiens) | KLK2 | 3817 | 19q13.2-q13.4 |
| S01.162 | kallikrein 3 | KLK3 | 354 | 19q13.3-q13.4 |
| S01.174 | mesotrypsin | PRSS3 | 5646 | 9p13 |
| S01.191 | complement factor D | DF | 1675 | 19 |
| S01.192 | complement component activated C1r | C1R | 715 | 12p13 |
| S01.193 | complement component activated C1s | C1S | 716 | 12p13 |
| S01.194 | complement component 2 | C2 | 717 | 6p21.3 |
| S01.196 | complement factor B | BF | 629 | 6p21.3 |
| S01.198 | mannan-binding lectin-associated serine protease 1 | MASP1 | 5648 | 3q27-q28 |
| S01.199 | complement factor I | IF | 3426 | 4q24-q25 |
| S01.205 | pancreatic endopeptidase E form B (B) | ELA3B | 23436 | 1p36.12 |
| S01.206 | pancreatic elastase II form B (Homo sapiens) (IIB) | | 51032 | 12q13 |
| S01.211 | coagulation factor XIIa | F12 | 2161 | 5q33-qter |
| S01.212 | plasma kallikrein | KLKB1 | 3818 | 4q35 |
| S01.213 | coagulation factor XIa | F11 | 2160 | 4q35 |
| S01.214 | coagulation factor IXa | F9 | 2158 | Xq27.1-q27.2 |
| S01.215 | coagulation factor VIIa | F7 | 2155 | 13q34 |
| S01.216 | coagulation factor Xa | F10 | 2159 | 13q34 |
| S01.217 | thrombin | F2 | 2147 | 11p11-q12 |
| S01.218 | protein C (activated) | PROC | 5624 | 2q13-q14 |
| S01.223 | acrosin | ACR | 49 | 22q13-qter |
| S01.224 | hepsin | HPN | 3249 | 19q11-q13.2 |
| S01.228 | hepatocyte growth factor activator | HGFAC | 3083 | 4p16 |
| S01.229 | mannan-binding lectin-associated serine protease 2 | MASP2 | 10747 | 1p36.3-p36.2 |
| S01.231 | u-plasminogen activator | PLAU | 5328 | 10q24 |
| S01.232 | t-plasminogen activator | PLAT | 5327 | 8p12 |
| S01.233 | plasmin | PLG | 5340 | 6q26 |
| S01.236 | neurosin | KLK6 | 5653 | 19q13.3-q13.4 |
| S01.237 | neurotrypsin | PRSS12 | 8492 | 4q25-q26 |
| S01.242 | tryptase beta 2 (Homo sapiens) (I) | TPSB1 | 7177 | 16p13.3 |
| S01.242 | tryptase beta 2 (Homo sapiens) (2) | TPSB2 | 64499 | 16p13.3 |
| S01.244 | neuropsin | KLK8 | 11202 | 19q13.3-q13.4 |
| S01.246 | kallikrein hK10 (Homo sapiens) | KLK10 | 5655 | 19q13.33 |
| S01.247 | epitheliasin | TMPRSS2 | 7113 | 21q22.3 |
| S01.251 | prostase | KLK4 | 9622 | 19q13.3-q13.4 |
| S01.252 | brain serine proteinase 2 | | 64063 | 16p13.3 |
| S01.256 | chymopasin | CTRL | 1506 | 16q22.1 |
| S01.257 | kallikrein 11 | KLK11 | 11012 | 19q13.3-q13.4 |
| S01.258 | anionic trypsin (Homo sapiens) (II) | PRSS2 | 5645 | 7q35 |
| S01.291 | LOC144757 peptidase (Homo sapiens) | | | 12q13.13 |
| S01.292 | Mername-AA169 peptidase | | | 4q13.1 |
| S01.294 | Mername-AA171 peptidase | | | |
| S01.298 | Mername-AA174 peptidase | | 154754 | 7q34 |
| S01.299 | Mername-AA175 peptidase | | | 8p23.1 |
| S01.300 | stratum corneum chymotryptic enzyme | KLK7 | 5650 | 19q13.3-q13.4 |
| S01.301 | trypsin-like enzyme, respiratory (Homo sapiens) | | 9407 | 4q13.2 |
| S01.302 | matriptase | ST14 | 6768 | 11q24-q25 |
| S01.306 | kallikrein hK13 | KLK13 | 26085 | 19q19.3-q19.4 |
| S01.307 | kallikrein hK9 (human numbering) | KLK9 | 23579 | 19q19.3-q19.4 |
| S01.308 | Mername-AA035 peptidase | | 164656 | 22q13.1 |
| S01.309 | umbelical vein proteinase | | 11098 | 11q14.1 |
| S01.311 | LCLP proteinase (LCLP (N-terminus)) | | | |
| S01.313 | spinesin | TMPRSS5 | 80975 | 11q23.3 |
| S01.318 | Mername-AA178 peptidase | | | 1 |
| S01.320 | Mername-AA180 peptidase | | | 11p15.3 |
| S01.322 | Mername-AA182 peptidase | | | 12p12.1 |
| S01.414 | Mername-AA122 peptidase (deduced from ESTs by MEROPS) | | | |

### Engineering proteases.

Virtually every aspect of a protease can be re-engineered, including the enzyme substrate sequence specificity, thermostability, pH profile, catalytic efficiency, oxidative stability, and catalytic function.

Existing proteases are used as scaffolds which include various mutations which change their substrate specificity. Scaffolds can largely include the amino acid sequences of trypsin, chymotrypsin, substilisin, thrombin, plasmin, Factor Xa, urokinase type plasminogen activator (uPA), tissue plasminogen activator (tPA), granzyme B, elastase, papain, cruzain, membrane type serine protease-1 (MTSP-1), chymase, neutrophil elastase, granzyme A, plasma kallikrein, granzyme M, complement factor serine proteases, ADAMTS13, neural endopeptidase/neprilysin, and furin or combinations thereof. Preferred scaffolds include granzyme B, MTSP-1, chymase, neutrophil elastase, granzyme A, plasma kallikrein, urokinase type plasminogen activator, granzyme M, chymotrypsin, thrombin, complement factor serine proteases, ADAMTS 13, neural endopeptidase/neprilysin, furin, and plasmin. Determinants of substrate sequence specificity in serine proteases come from the S1-S4 positions in the active site, where the protease is in contact with the P1-P4 residues of the peptide substrate sequence. In some cases, there is little (if any) interaction between the S 1-S4 pockets of the active site, such that each pocket appears to recognize and bind the corresponding residue on the peptide substrate sequence independent of the other pockets. Thus the specificity determinants may be generally changed in one pocket without affecting the specificity of the other pockets.

For example, a protease with low specificity for a residue at a particular binding site or for a particular sequence is altered in its specificity by making point mutations in the substrate sequence binding pocket. In some cases, the resulting mutant has a greater than 10-fold increase in specificity at a site or for a particular sequence than does wild-type. In another embodiment, the resulting mutant has a greater than 100-fold increase in specificity at a site or for a particular sequence than does wild-type. In another embodiment, the resulting mutant has an over 1000-fold increase in specificity at a site or for a particular sequence than does wild-type.

Also contemplated by the invention are libraries of scaffolds with various mutations that are generated and screened using methods known in the art and those detailed below. Libraries are screened to ascertain the substrate sequence specificity of the members. Libraries of scaffolds are tested for specificity by exposing the members to substrate peptide sequences. The member with the mutations that allow it to cleave the substrate sequence is identified. The library is constructed with enough variety of mutation in the scaffolds that any substrate peptide sequence is cleaved by a member of the library. Thus, proteases specific for any target protein can be generated.

### The Process

### 1. Choosing A Scaffold

In another embodiment of the invention, scaffold proteases are chosen using the following requirements: 1) The protease is a human or mammalian protease of known sequence; 2) the protease can be manipulated through current molecular biology techniques; 3) the protease can be expressed heterologously at relatively high levels in a suitable host; and 4) the protease can be purified to a chemically competent form at levels sufficient for screening. In other embodiments of the invention, the scaffold protease to be mutated cleaves a protein that is found extracellularly. This extracellular protein is, for example, a receptor, a signaling protein, or a cytokine. The residues that, upon mutation, affect the activity and specificity of two families of scaffold proteases are described here. Preferably, there is three dimensional structural information for the protease is available. Also, it is preferred that there be knowledge of the initial substrate specificity of the protease. It is also preferable that the protease be active and stable *in vitro* and that knowledge of macromolecular modulators of the protease are available. Also, proteases are preferred which cleave targets that are relevant to affecting pathology, e.g. inactivating protein effectors of pathology.

### Serine Proteases.

In another embodiment of the invention, serine proteases with altered specificity are generated by a structure-based design approach. Each protease has a series of amino acids that line the active site pocket and make direct contact with the substrate. Throughout the chymotrypsin family, the backbone interaction between the substrate and enzyme are completely conserved, but the side chain interactions vary considerably throughout the family. The identity of the amino acids that comprise the S1-S4 pockets of the active site determines the substrate specificity of that particular pocket. Grafting the amino acids of one serine protease to another of the same fold modifies the specificity of one to the other. For example, a mutation at position 99 in the S2 pocket to a small amino acid confers a preference for large hydrophobic residues in the P2 substrate position. Using this process of selective mutagenesis, followed by substrate library screening, proteases are designed with novel substrate specificities towards proteins involved with various diseases.

The serine proteases are members of the same family as chymotrypsin, in that they share sequence and structural homology with chymotrypsin. The active site residues are Asp102, His 57, and Ser 195. The linear amino acid sequence can be aligned with that of chymotrypsin and numbered according to the β sheets of chymotrypsin. Insertions and deletions occur in the loops between the beta sheets, but throughout the structural family, the core sheets are conserved. The serine proteases interact with a substrate in a conserved β sheet manner. Up to 6 conserved hydrogen bonds can occur between the substrate and the enzyme.

### Cysteine Proteases

Papain-like cysteine proteases are a family of thiol dependent endo-peptidases related by structural similarity to papain. They form a two-domain protein with the domains labeled R and L (for right and left) and loops from both domains form a substrate recognition cleft. They have a catalytic triad made up of the amino acids Cys 25, His 159 and Asn 175. Unlike serine proteases (which recognize and proteolyze a target peptide based on a β-sheet conformation of the substrate), this family of proteases does not have well-defined pockets for substrate recognition. The main substrate recognition occurs at the P2 amino acid, (compared to the P1 residue in serine proteases).

The S2 pocket is the most selective and best characterized of the protease substrate recognition sites. It is defined by the amino acids at the following spatial positions (papain numbering): 66, 67, 68, 133, 157, 160 and 205. Position 205 plays a role similar to position 189 in the serine proteases- a residue buried at the bottom of the pocket that determines the specificity.

The substrate specificity of a number of cysteine proteases (human cathepsin L, V, K, S, F, B, papain, and cruzain) were profiled using a complete diverse positional scanning synthetic combinatorial library (PS-SCL). The complete library consists of P1, P2, P3, and P4 tetrapeptide substrates in which one position is held fixed while the other three positions are randomized with equal molar mixtures of the 20 possible amino acids, giving a total diversity of ~160,000 tetrapeptide sequences.

Overall, P1 specificity was almost identical between the cathepsins, with Arg and Lys being strongly favored while small aliphatic amino acids were tolerated. Much of the selectivity was found in the P2 position, where the human cathepsins were strictly selective for hydrophobic amino acids. Interestingly, P2 specificity for hydrophobic residues was divided between aromatic amino acids such as Phe, Tyr, and Trp (cathepsin L, V), and bulky aliphatic amino acids such as Val or Leu (cathepsin K, S, F). Compared to the P2 position, selectivity at the P3 position was significantly less stringent. However, several of the proteases showed a distinct preference for proline (cathepsin V, S, and papain), leucine (cathepsin B), or arginine (cathepsin S, cruzain). The proteases showed broad specificity at the P4 position, as no one amino acid was selected over others.

### Substrate recognition profiles

To make a variant protease with an altered substrate recognition profile, the amino acids in the three-dimensional structure that contribute to the substrate selectivity (specificity determinants) are targeted for mutagenesis. For the serine proteases, numerous structures of family members have defined the surface residues that contribute to extended substrate specificity (Wang et al., Biochemistry 2001 Aug 28;40(34):10038-46; Hopfner et al., Structure Fold Des. 1999 Aug 15;7(8):989-96; Friedrich et al. J Biol Chem. 2002 Jan 18;277(3):2160-8; Waugh et al., Nat Struct Biol. 2000 Sep;7(9):762-5). Structural determinants for various proteases are listed in Table 3, along with a listing of the amino acid in a subset of family members determined to be of known, extended specificity. For serine proteases, the following amino acids in the primary sequence are determinants of specificity: 195, 102, 57 (the catalytic triad); 189, 190,191, 192, and 226 (P1); 57, the loop between 58 and 64, and 99 (P2); 192,217,218 (P3), the loop between Cys168 and Cys180, 215 and 97 to 100 (P4).

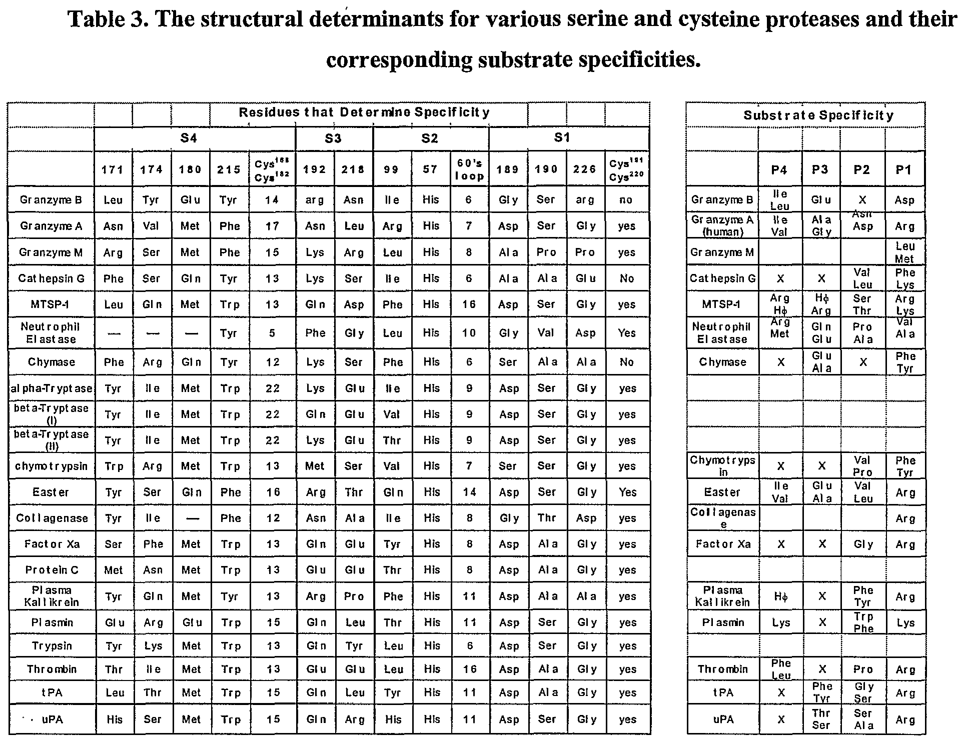

Granzyme B is a member of the family of chymotrypsin fold serine proteases, and has greater than 50% identity to other members of the granzyme family including granzymes C-G, cathepsin G, and rat mast cell protease II. The protein is a sandwich of two six stranded, anti-parallel β-barrel domains connected by a short α-helix. The catalytic triad is composed of Asp102, His 57 and Ser 195. The surface loops are numbered according to the additions and deletions compared to α-chymotrypsin and represent the most variable regions of this structural family. The determinants of specificity are defined by the three-dimensional structure of rat granzyme B in complex with ecotin [IEPD], a macromolecular inhibitor with a substrate-like binding loop (Waugh et al., Nature Struct. Biol). These structural determinants of specificity include Ile99, Arg192, Asn218, Tyr215, Tyr174, Leu172, Arg226, and Tyr 151, by chymotrypsin numbering. Interestingly, the other members of the granzyme family of serine proteases share only two of these amino acids with granzyme B. They are Tyr 215 and Leu 172, two residues that vary very little across the entire structural family. This suggests that while the sequence identity of the granzymes is high, their substrate specificities are very different.

To determine the role of these amino acids in extended specificity, Ile99, Arg192, Asn218 and Tyr174 were mutated to the amino acid alanine. It was determined that Ile99 contributes to P2 specificity, Asn218 and Arg192 to P3 specificity, and Tyr174 to P4 specificity. Each modified protease was profiled using a combinatorial substrate library to determine the effect of the mutation on extended specificity. Since the P1 specificity of a protease represents the majority of its specificity, the modifications do not destroy unique specificity of granzyme B towards P1 aspartic acid amino acids but modulate specificity in the extended P2 to P4 sites.

For the P3 and P4 subsites, mutations at Tyr174, Arg192 and Asn218 did not significantly affect the specificity (*See* Table 4, below). Y174A increases the activity towards Leu at P4, but the rest of the amino acids continue to be poorly selected. R192A and N218A both broaden the specificity at P3. Instead of a strong preference for glutamic acid, Ala, Ser, Glu and Gln are similarly preferred in the mutant. The overall activity (kcat/Km) of the mutant is less than 10% below the wild type activity toward an ideal wild-type substrate, N-acetyl-Ile-Glu-Pro-Asp-AMC (7-amino-4-methylcoumarin) (Ac-IEPD-AMC).

A much more dramatic effect is observed at the P2 subsite (*See* Table 4, below). In wild type granzyme B, the preference is broad with a slight preference for Pro residues. I99A narrows the P2 specificity to Phe and Tyr residues. Phe is now preferred nearly 5 times over the average activity of other amino acids at this position. Within the chymotrypsin family of serine proteases, more than a dozen proteases have a small residue at this structural site, either an asparagine, serine, threonine, alanine or glycine. From this group, two proteases have been profiled using combinatorial substrate libraries, (plasma kallikrein and plasmin), and both show strong preferences towards Phe and Tyr. These two results suggest that any serine protease that is mutated to an Asn, Ser, Thr, Gly or Ala at position 99 will show the same hydrophobic specificity found in plasma kallikrein, plasmin and the I99A granzyme B mutant.

The understanding of the P2 specificity determinants may be expanded to the contrasting mutation and substrate preference. Nearly two dozen chymotrypsin-fold serine proteases have an aromatic amino acid at position 99. Four of these proteases have been profiled using combinatorial substrate libraries: human granzyme B, tissue type plasminogen activator, urokinase type plasminogen activator, and membrane type serine protease 1. All but granzyme B have a preference for serine, glycine and alanine amino acids at the substrate P2 position.

**Table 4. Granzyme B Mutations**

| | **S4** | | | | | **S3** | | **S2** | | | **S1** | | | |
|---|---|---|---|---|---|---|---|---|---|---|---|---|---|---|
| **Mutant** | **171** | **174** | **180** | **215** | **Cys 168-182** | **192** | **218** | **99** | **57** | **60's Loop** | **189** | **190** | **226** | **Cys 191-220** |
| Wildtype | Leu | Tyr | Glu | Tyr | 14 | Arg | Asn | Ile | His | 6 | Gly | Ser | Arg | No |
| I99F | Leu | Tyr | Glu | Tyr | 14 | Arg | Asn | Phe | His | 6 | Gly | Ser | Arg | No |
| I99A | Leu | Tyr | Glu | Tyr | 14 | Arg | Asn | Ala | His | 6 | Gly | Ser | Arg | No |
| I99K | Leu | Tyr | Glu | Tyr | 14 | Arg | Asn | Lys | His | 6 | Gly | Ser | Arg | No |
| N218A | Leu | Tyr | Glu | Tyr | 14 | Arg | Ala | Ile | His | 6 | Gly | Ser | Arg | No |
| N218T | Leu | Tyr | Glu | Tyr | 14 | Arg | Thr | Ile | His | 6 | Gly | Ser | Arg | No |
| N218V | Leu | Tyr | Glu | Tyr | 14 | Arg | Val | Ile | His | 6 | Gly | Ser | Arg | No |
| R192A | Leu | Tyr | Glu | Tyr | 14 | Ala | Asn | Ile | His | 6 | Gly | Ser | Arg | No |
| R192E | Leu | Tyr | Glu | Tyr | 14 | Glu | Asn | Ile | His | 6 | Gly | Ser | Arg | No |
| Y174A | Leu | Ala | Glu | Tyr | 14 | Arg | Asn | Ile | His | 6 | Gly | Ser | Arg | No |
| Y174V | Leu | Val | Glu | Tyr | 14 | Arg | Asn | Ile | His | 6 | Gly | Ser | Arg | No |
| I99A/N21 8A | Leu | Tyr | Glu | Tyr | 14 | Arg | Ala | Ala | His | 6 | Gly | Ser | Arg | No |
| R192A/N 218A | Leu | Tyr | Glu | Tyr | 14 | Ala | Ala | Ile | His | 6 | Gly | Ser | Arg | No |
| R192E/N 218A | Leu | Tyr | Glu | Tyr | 14 | Glu | Ala | Ile | His | 6 | Gly | Ser | Arg | No |

**Table 5. Effects on Specificity**

| **Specificity Profile** | | | | |
|---|---|---|---|---|
| **Mutant** | **P4** | **P3** | **P2** | **P1** |
| Wildtype | Ile/Val | Glu | X | Asp |
| I99F | Ile/Val | Glu | X | Asp |
| I99A | Ile/Val | Glu | **Phe** | Asp |
| I99K | Ile/Val | Glu | X | Asp |
| N218A | Ile/Val | X | X | Asp |
| N218T | Ile/Val | **Ala/Ser** | X | Asp |
| N218V | Ile/Val | X | X | Asp |
| R192A | Ile/Val | Glu | X | Asp |
| R192E | Ile/Val | Lys/ Gln/ Ser | X | Asp |
| Y174A | **Ile/Val/Leu** | Glu | X | Asp |
| Y174V | Ile/Val | Glu | X | Asp |
| I99A/N21 8A | Phe/ Leu | Ala/ Ser | **Phe** | Asp |
| | **/Ile/Val** | | | |
| R192A/N 218A | Ile/Val | **Ala/Gln/Ser** | X | Asp |
| R192E/N 218A | Ile/Val | **ArgLysAla** | X | Asp |

From Tables 4 and 5, the determinants of specificity selected to be altered in rat granzyme B are as follows: Ser195, Asp102, His 57, Ala189, Ser190, Phe191, Arg192, Arg226, Ser 58, Gly59, Ser60, Lys61, Ile62, Asn63, Ile99, Gln217, Asn218, Glu169, Ser170, Tyr171, Leu171A (note the one amino acid insertion as compared to chymotrypsin), Lys172, Asn173, Tyr174, Phe175, Asp176, Lys177, Ala178, Asn179, Glu180, Ile181, Tyr215, Lys97, Thr98, Ile99, and Ser100.

For the cysteine proteases, the amino acids selected to be modified are less well described. The S2 pocket is the most selective and best characterized of the protease substrate recognition sites. It is defined by the amino acids at the following three-dimensional positions (papain numbering): 66, 67, 68, 133, 157, 160 and 205. Position 205 plays a role similar to position 189 in the serine proteases- a residue buried at the bottom of the pocket that determines the specificity. The other specificity determinants include the following amino acids (numbered according to papain): 61 and 66 (P3); 19, 20, and 158 (P1).

**Table 6. The structural determinants for various cysteine proteases and their corresponding substrate specificities.**

| | | | | **Residues that Determine Specificity** | | | | | | | | | | | **Substrate Specificity** | | | | |
|---|---|---|---|---|---|---|---|---|---|---|---|---|---|---|---|---|---|---|---|
| | **Active Site Residues** | | | **S3** | | **S2** | | | | | **S1** | | | | | | | | |
| | **25** | **159** | **175** | **61** | **66** | **66** | **133** | **157** | **160** | **205** | **19** | **20** | **158** | | | **P4** | **P3** | **P2** | **P1** |
| Cathepsin L | Cys | His | Asn | Glu | Gly | Gly | Ala | Met | Gly | Ala | Gln | Gly | Asp | | Cathepsin L | X | X | Phe Trp | Arg Lys |
| Cathepsin V | Cys | His | Asn | Gln | Gly | Gly | Ala | Leu | Gly | Ala | Gln | Lys | Asp | | Cat hepsin V | X | Pro X | Trp Tyr Phe | X |
| Cathepsin K | Cys | His | Asn | Asp | Gly | Gly | Ala | Leu | Ala | Leu | Gln | Gly | Asn | | Cathepsin K | X | X | Leu Pro | Arg Lys |
| Cathepsin S | Cys | His | Asn | Lys | Gly | Gly | Gly | Val | Gly | Phe | Gln | Gly | Asn | | Cathepsin S | X | Arg X | Val Leu Met | Lys Arg |
| Cathepsin F | Cys | His | Asn | Lys | Gly | Gly | Ala | Ile | Ala | Met | Gln | Gly | Asp | | Cathepsin F | X | X | Leu | Lys Arg |
| Cathepsin B | Cys | His | Asn | Asp | Gly | Gly | Ala | Gly | Ala | Glu | Gln | Gly | Gly | | Cathepsin B | X | Leu X | Val | Arg Lys |
| Papain | Cys | His | Asn | Tyr | Gly | Gly | Val | Val | Alaa | Ser | Gln | Gly | Asp | | Papain | X | Pro X | Val Phe Lyr | Arg Lys |
| Cruzain | Cys | His | Asn | Ser | Gly | Gly | Ala | Leu | Gly | Glu | Gln | Gl y | Asp | | Cruzain | X | Arg X | Leu Phe Lyr | Arg Lys |

### 2. Mutagenesis Of The Scaffold Protease

In order to change the substrate preference of a given subsite (S1-S4) for a given amino acid, the specificity determinants that line the binding pocket are mutated, either individually or in combination. In one embodiment of the invention, a saturation mutatgenesis technique is used in which the residue(s) lining the pocket is mutated to each of the 20 possible amino acids. This can be accomplished using the Kunkle method (Current Protocols in Molecular Biology, John Wiley and Sons, Inc., Media Pa.). Briefly, a mutagenic oligonucleotide primer is synthesized which contains either NNS or NNK-randomization at the desired codon. The primer is annealed to the single stranded DNA template and DNA polymerase is added to synthesize the complementary strain of the template. After ligation, the double stranded DNA template is transformed into *E*. *coli* for amplification. Alternatively, single amino acid changes are made using standard, commercially available site-directed mutagenesis kits such as QuikChange (Stratagene). In another embodiment, any method commonly known in the art for site specific amino acid mutation could be used.

### 3. Express And Purify The Variant Protease

The protease may be expressed in an active or inactive, zymogen form. The protease may be in a heterologously expressing system such as *E. coli, Pichia pastoris, S. cerevisae,* or a baculovirus expression system. The protein can either be expressed in an intracellular environment or excreted into the media. The protease can also be expressed in an *in vitro* expression system. To purify the variant protease, column chromatography can be used. The protease may contain an C-terminal 6-His tag for purification on a Nickel column. Depending on the pI of the protease, a cation or anion exchange column may be appropriate. The protease can be stored in a low pH buffer that minimizes its catalytic activity so that it will not degrade itself. Purification can also be accomplished through immunoabsorption, gel filtration, or any other purification method commonly used in the art.

### 4. Synthesis Of ACC Positional Scanning Libraries

Those of skill in the art will recognize that many methods can be used to prepare the peptides and the libraries of the invention. In an exemplary embodiment, the library is screened by attaching a fluorogenically tagged substrate peptide to a solid support. The fluorogenic leaving group from substrate peptide is synthesized by condensing an N-Fmoc coumarin derivative, to acid-labile Rink linker to provide ACC resin (Backes, et al. Nat Biotechnol. 2000 Feb;18(2):187-93). Fmoc-removal produces a free amine. Natural, unnatural and modified amino acids can be coupled to the amine, which can be elaborated by the coupling of additional amino acids. After the synthesis of the peptide is complete, the peptide-fluorogenic moiety conjugate can be cleaved from the solid support or, alternatively, the conjugate can remain tethered to the solid support.

Thus, in a further preferred embodiment, the present invention provides a method of preparing a fluorogenic peptide or a material including a fluorogenic peptide. The method includes: (a) providing a first conjugate comprising a fluorogenic moiety covalently bonded to a solid support; (b) contacting the first conjugate with a first protected amino acid moiety and an activating agent, thereby forming a peptide bond between a carboxyl group and the amine nitrogen of the first conjugate; (c) deprotecting, thereby forming a second conjugate having a reactive amine moiety; (d) contacting the second conjugate with a second protected amino acid and an activating agent, thereby forming a peptide bond between a carboxyl group and the reactive amine moiety; and (e) deprotecting, thereby forming a third conjugate having a reactive amine moiety.

In a preferred embodiment, the method further includes: (f) contacting the third conjugate with a third protected amino acid and an activating agent, thereby forming a peptide bond between a carboxyl group and the reactive amine moiety; and (e) deprotecting, thereby forming a fourth conjugate having a reactive amine moiety.

For amino acids that are difficult to couple (Ile, Val, etc), free, unreacted amine may remain on the support and complicate subsequent synthesis and assay operations. A specialized capping step employing the 3-nitrotriazole active ester of acetic acid in DMF efficiently acylates the remaining aniline. The resulting acetic acid-capped coumarin that may be present in unpurified substrate sequence solutions is generally not a protease substrate sequence. P1-substituted resins that are provided by these methods can be used to prepare any ACC-fluorogenic substrate.

In a further preferred embodiment, diversity at any particular position or combination of positions is introduced by utilizing a mixture of at least two, preferably at least 6, more preferably at least 12, and more preferably still, at least 20, amino acids to grow the peptide chain. The mixtures of amino acids can include of any useful amount of a particular amino acid in combination with any useful amount of one or more different amino acids. In a presently preferred embodiment, the mixture is an isokinetic mixture of amino acids (a mixture in appropriate ratios to allow for equal molar reactivity of all components). An isokinetic mixture is one in the molar ratios of amino acids has been adjusted based on their reported reaction rates. (Ostresh, J. M., Winkle, J. H., Hamashin, V. T., & Houghten, R. A. (1994). Biopolymers 34, 1681-1689).

Solid phase peptide synthesis in which the C-terminal amino acid of the sequence is attached to an insoluble support followed by sequential addition of the remaining amino acids in the sequence is the preferred method for preparing the peptide backbone of the compounds of the present invention. Techniques for solid phase synthesis are described by Barany and Merrifield, Solid-Phase Peptide Synthesis; pp. 3-284 in The Peptides: Analysis, Synthesis, Biology. Vol. 2; Special Methods In Peptide Synthesis, Part A., Gross and Meienhofer, eds. Academic press, N.Y., 1980; and Stewart et al., Solid Phase Peptide Synthesis, 2nd ed. Pierce Chem. Co., Rockford, III. (1984) which are incorporated herein by reference. Solid phase synthesis is most easily accomplished with commercially available peptide synthesizers utilizing Fmoc or t-BOC chemistry.

In a particularly preferred embodiment, peptide synthesis is performed using Fmoc synthesis chemistry. The side chains of Asp, Ser, Thr and Tyr are preferably protected using t-butyl and the side chain of Cys residue using S-trityl and S-t-butylthio, and Lys residues are preferably protected using t-Boc, Fmoc and 4-methyltrityl. Appropriately protected amino acid reagents are commercially available or can be prepared using art-recognized methods. The use of multiple protecting groups allows selective deblocking and coupling of a fluorophore to any particular desired side chain. Thus, for example, t-Boc deprotection is accomplished using TFA in dichloromethane. Fmoc deprotection is accomplished using, for example, 20% (v/v) piperidine in DMF or N-methylpyrolidone, and 4-methyltrityl deprotection is accomplished using, for example, 1 to 5% (v/v) TFA in water or 1% TFA and 5% triisopropylsilane in DCM. S-t-butylthio deprotection is accomplished using, for example, aqueous mercaptoethanol (10%). Removal of t-butyl, t-boc and S-trityl groups is accomplished using, for example, TFA:phenol:water:thioanisol:ethanedithiol (85:5:5:2.5:2.5), or TFA:phenol:water (95:5:5).

### 5. Screen The Protease For Specificity Changes.

Essential amino acids in the proteases generated using the methods of the present invention are identified according to procedures known in the art, such as site-directed mutagenesis or saturation mutagenesis of active site residues. In the latter technique, residues that form the S1-S4 pockets that have been shown to be important determinants of specificity are mutated to every possible amino acid, either alone or in combination. See for example, Legendre, et al., JMB (2000) 296: 87-102. Substrate specificities of the resulting mutants will be determined using the ACC positional scanning libraries and by single substrate kinetic assays (Harris, et al. PNAS, 2000, 97:7754-7759).

Multiple amino acid substitutions are made and tested using known methods of mutagenesis and screening, such as those disclosed by Reidhaar-Olson and Sauer (Science 241:53-57, 1988) or Bowie and Sauer (Proc. Natl. Acad. Sci. USA 86:2152-2156, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (*e.g*., Legendre et al., JMB, 2000: 296:87-102; Lowman et al., Biochem. 30:10832-10837, 1991; Ladner et al., U.S. Pat. No. 5,223,409; Huse, PCT Publication WO 92/06204) and region-directed mutagenesis (Derbyshire et al., Gene 46:145, 1986; Ner et al., DNA 7:127, 1988).

Mutagenesis methods as disclosed above can be combined with high-throughput, automated screening methods to detect activity of cloned, mutagenized polypeptides in host cells. Mutagenized DNA molecules that encode proteolytically active proteins or precursors thereof are recovered from the host cells and rapidly sequenced using modem equipment. These methods allow the rapid determination of the importance of individual amino acid residues in a polypeptide of interest, and can be applied to polypeptides of unknown structure.

### Screening by Protease Phage Display

In one embodiment protease phage display is used to screen pools of mutant proteases for various affinities to specific substrate sequences as described in Legendre et al., JMB, 2000: 296:87-102, and Corey et al., Gene, 1993 Jun 15;128(1):129-34. The phage technique allows one to provide a physical link between a protein and the genetic information encoding it. The protein of interest is constructed as a genetic fusion to a surface coat protein of a bacterial virus. When the viral particle is produced in a bacterial host, the protein of interest is produced as a fusion protein and displayed on the surface of the virus, and its gene is packed within the capsid particle of the virus. Phage-displayed random protein libraries are screened for binding to immobilized targets. Libraries of phage (with each phage representing an individual mutant) are sorted for enhanced affinity against the target. Serine proteases have been displayed on the surface of phage and this technique, coupled with a suitable mutagenesis technique, is used to generate a diverse library of protease variants.

The target which is selected may be one related to a therapeutic application of the protease. For example, the target sequence is present in an endotoxin, or a viral protein, or a bacterial wall protein, or a native blood-born peptide related to an auto-immune condition. Here the protease selected is used in a treatment method, by administering the peptide, *e.g*., by intravenous administration, to a person in need of such treatment.

### Screening Using Fluorescence

In another embodiment of the invention, a method of assaying for the presence of an enzymatically active protease. The method includes: (a) contacting a sample with a protease, in such a manner whereby a fluorogenic moiety is released from a peptide substrate sequence upon action of the protease, thereby producing a fluorescent moiety; and (b) observing whether the sample undergoes a detectable change in fluorescence, the detectable change being an indication of the presence of the enzymatically active protease in the sample.

This method of the invention can be used to assay for substantially any known or later discovered protease. The sample containing the protease can be derived from substantially any source, or organism. In one embodiment, the sample is a clinical sample from a subject. In another embodiment, the protease is a member selected from the group consisting of aspartic protease, cysteine protease, metalloprotease and serine protease. The method of the invention is particularly preferred for the assay of proteases derived from a microorganism, including, but not limited to, bacteria, fungi, yeast, viruses, and protozoa.

Assaying for protease activity in a solution simply requires adding a quantity of the stock solution to a fluorogenic protease indicator and measuring the subsequent increase in fluorescence or decrease in excitation band in the absorption spectrum. The solution and the fluorogenic indicator may also be combined and assayed in a "digestion buffer" that optimizes activity of the protease. Buffers suitable for assaying protease activity are well known to those of skill in the art. In general, a buffer is selected with a pH which corresponds to the pH optimum of the particular protease. For example, a buffer particularly suitable for assaying elastase activity consists of 50 mM sodium phosphate, 1 mM EDTA at pH 8.9. The measurement is most easily made in a fluorometer, an instrument that provides an "excitation" light source for the fluorophore and then measures the light subsequently emitted at a particular wavelength. Comparison with a control indicator solution lacking the protease provides a measure of the protease activity. The activity level may be precisely quantified by generating a standard curve for the protease/indicator combination in which the rate of change in fluorescence produced by protease solutions of known activity is determined.

While detection of the fluorogenic compounds is preferably accomplished using a fluorometer, detection may be accomplished by a variety of other methods well known to those of skill in the art. Thus, for example, when the fluorophores emit in the visible wavelengths, detection may be simply by visual inspection of fluorescence in response to excitation by a light source. Detection may also be by means of an image analysis system utilizing a video camera interfaced to a digitizer or other image acquisition system. Detection may also be by visualization through a filter, as under a fluorescence microscope. The microscope may provide a signal that is simply visualized by the operator. Alternatively, the signal may be recorded on photographic film or using a video analysis system. The signal may also simply be quantified in real time using either an image analysis system or a photometer.

Thus, for example, a basic assay for protease activity of a sample involves suspending or dissolving the sample in a buffer (at the pH optima of the particular protease being assayed), adding to the buffer a fluorogenic protease indicators, and monitoring the resulting change in fluorescence using a spectrofluorometer as shown in Harris et al., J Biol Chem, Vol. 273, Issue 42, 27364-27373, October 16, 1998. The spectrofluorometer is set to excite the fluorophore at the excitation wavelength of the fluorophore and to detect the resulting fluorescence at the emission wavelength of the fluorophore. The fluorogenic protease indicator is a substrate sequence of a protease that changes in fluorescence due to a protease cleaving the indicator.

In an illustrative embodiment, the invention provides a library useful for profiling of various serine and cysteine proteases. The library is able to distinguish proteases having specificity for different amino acids.

In another illustrative embodiment, a library is provided for probing the extended substrate sequence specificity of several serine proteases involved in blood coagulation, in which the P1 position is held constant as either Lys or Arg, depending on the preferred P1-specificity of the protease.

The PS-SCL strategy allows for the rapid and facile determination of proteolytic substrate sequence specificity. Those of skill in the art will appreciate that these methods provide a wide variety of alternative library formats. For example, fixing the P2-position as a large hydrophobic amino acid may circumvent preferential internal cleavage by papain-fold proteases and lead to proper register of the substrate sequence. Determination and consideration of particular limitations relevant to any particular enzyme or method of substrate sequence specificity determination are within the ability of those of skill in the art.

In addition to use in assaying for the presence of a selected enzyme, the method of the invention is also useful for detecting, identifying and quantifying an enzyme in a sample (*e.g*., protease). Thus, in another preferred embodiment, the screening method further includes, (c) quantifying the fluorescent moiety, thereby quantifying the enzyme (*e.g.* protease) present in the sample. The sample can be, *e.g*. a biological fluid, such as blood, serum, urine, tears, milk or semen

### Screening Using Protease Sequence Specificity Assay

In another preferred embodiment, these methods are used select for an enzyme that specifically cleaves a target sequence, and preferably for an enzymatically active protease. The method includes: (a) a random peptide library containing an internally quenched fluorophore, where the fluorophore is *e.g*. o-aminobenzoyl and the quencher is *e.g*. 3-nitrotyrosine; (b) a peptide substrate sequence corresponding to the sequence targeted for cleavage, which also contains an internally quenched fluorophore where the fluorophore is *e.g.* Cy3B and the quencher is *e.g.* Cy5Q; (c) mixing the random peptide library and peptide substrate sequence at a 1:1 ratio; (d) exposing the mixture to the mutant protease and then quantitating the ratio of Cy3B fluorescence to o-aminobenzoyl fluorescence. If a protease is selective for the target peptide, it will cleave only the target peptide and not the random library, and thus there will be a high ratio of Cy3B fluorescence to o-aminobenzoyl fluorescence. (Meldal and Breddam, Anal. Biochem. (1991) 195: 141-147; Gron, et al. Biochemistry (1992) 31: 6011-6018)

In another preferred embodiment, these methods are used to determine the sequence specificity of an enzyme, and preferably of an enzymatically active protease. The method includes: (a) contacting the protease with a library of peptides of the invention in such a manner whereby the fluorogenic moiety is released from the peptide sequence, thereby forming a fluorescent moiety; (b) detecting the fluorescent moiety; and (c) determining the sequence of the peptide sequence, thereby determining the peptide sequence specificity profile of the protease.

In a preferred embodiment of the above-described method, the method further includes, (d) quantifying the fluorescent moiety, thereby quantifying the protease.

Moreover, in each of the aspects and embodiments set forth hereinabove, the protease can be substantially any protease of interest, but is preferably aspartic protease, cysteine protease, metalloprotease or serine protease. The protease assayed using a method of the invention can be derived from substantially any organism, including, but not limited to, mammals (*e.g*. humans), birds, reptiles, insects, plants, fungi and the like. In a preferred embodiment, the protease is derived from a microorganism, including, but not limited to, bacteria, fungi, yeast, viruses, and protozoa.

### 6. Iteration Of Steps 1-5

The method is repeated iteratively to create a variant protease that has the desired specificity and selectivity at each of the extended binding subsites, P2, P3, and P4. In some cases, mutations in serine proteases have shown that each of the subsites that form the active site (S 1-S4) function independently of one another, such that modification of specificity at one subsite has little influence on specificity at adjacent subsites. Thus, engineering substrate specificity and selectivity throughout the extended binding site can be accomplished in a step-wise manner.

Mutant proteases that match the desired specificity profiles, as determined by substrate libraries, are then assayed using individual peptide substrates corresponding to the desired cleavage sequence. Variant proteases are also assayed to ascertain that they will cleave the desired sequence when presented in the context of the full-length protein. The activity of the target protein is also assayed to verify that its function has been destroyed by the cleavage event. The cleavage event is monitored by SDS-PAGE after incubating the purified full-length protein with the variant protease.

In another embodiment, mutant proteases are combined to acquire the specificity of multiple proteases. A mutation at one residue of a scaffold, which produces specificity at one site, is combined in the same protease with another mutation at another site on the scaffold to make a combined specificity protease. Any number of mutations at discrete sites on the same scaffold can be used to create a combined specificity protease. In one specific embodiment, a mutation in the granzyme B scaffold at position 99 from isoleucine to alanine was combined with a mutation at position 218 of asparagine to alanine to create the combined specificity protease 199A/N218A granzyme B, the properties of which are detailed herein.

Proteins targeted for cleavage and inactivation are identified by the following criteria: 1) the protein is involved in pathology; 2) there is strong evidence the protein is the critical point of intervention for treating the pathology; 3) proteolytic cleavage of the protein will likely destroy its function. Cleavage sites within target proteins are identified by the following criteria: 1) they are located on the exposed surface of the protein; 2) they are located in regions that are devoid of secondary structure (*i.e*. not in β sheets or β helices), as determined by atomic structure or structure prediction algorithms; (these regions tend to be loops on the surface of proteins or stalks on cell surface receptors); 3) they are located at sites that are likely to inactivate the protein, based on its known function. Cleavage sequences are *e.g.*, four residues in length to match the extended substrate specificity of many serine proteases, but can be longer or shorter.

In another embodiment of the invention, target protein-assisted catalysis is used to generate proteases specific for a target protein. In target protein-assisted catalysis, the invariant histidine that is part of the catalytic triad in a serine protease is mutated to alanine, rendering the protease inactive. A histidine in the proper position in the target protein could function as a hydrogen acceptor, in effect playing the same role as the mutated histidine in the protease, thereby restoring catalytic activity. However, this places a stringent requirement for having a histidine in the proper position in the substrate sequence (P2 or P1'). A single mutation in the substrate sequence binding site of the protease can alter its specificity and cause it to have a change in substrate sequence specificity. Substrate sequence specificity can be altered using a small number of mutations.

Using the methods disclosed above, one of ordinary skill in the art can identify and/or prepare a variety of polypeptides that are substantially homologous to a protease scaffold or allelic variants thereof and retain the proteolytic properties of the wild-type protein. In one embodiment, these scaffolds comprise the amino acid sequences of trypsin, chymotrypsin, substilisin, thrombin, plasmin, Factor Xa, uPA, tPA, granzyme B, granzyme A, chymase, MTSP-1, cathepsin G, elastase, papain, or cruzain. Such polypeptides may include a targeting moiety comprising additional amino acid residues that form an independently folding binding domain. Such domains include, for example, an extracellular ligand-binding domain (e.g., one or more fibronectin type III domains) of a cytokine receptor; immunoglobulin domains; DNA binding domains (see, *e.g.,* He et al., Nature 378:92-96, 1995); affinity tags; and the like. Such polypeptides may also include additional polypeptide segments as generally disclosed above.

### Protease Polypeptides

A polypeptide according to the invention includes a polypeptide including the amino acid sequence of a protease whose sequence is provided in any one of the scaffolds described herein. The invention also includes a mutant or variant protease any of whose residues may be changed from the corresponding residues shown in any one of the scaffolds described herein, while still encoding a protein that maintains its protease activities and physiological functions, or a functional fragment thereof. In a preferred embodiment, the mutations occur in the S1-S4 regions of the protease as detailed herein.

In general, a protease variant that preserves protease-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated proteases, and biologically-active portions thereof, or derivatives, fragments, analogs or homologs thereof. Also provided are polypeptide fragments suitable for use as immunogens to raise anti-protease antibodies. In another embodiment, proteases are produced by recombinant DNA techniques. Alternative to recombinant expression, a protease protein or polypeptide can be synthesized chemically using standard peptide synthesis techniques.

Biologically-active portions of protease proteins include peptides comprising amino acid sequences sufficiently homologous to or derived from the amino acid sequences of the protease proteins that include fewer amino acids than the full-length protease proteins, and exhibit at least one activity of a protease protein. Typically, biologically-active portions comprise a domain or motif with at least one activity of the protease protein. A biologically-active portion of a protease protein is a polypeptide which is, for example, 10, 25, 50, 60, 70, 80, 90, 100, 110, 120, 130, 140, 150, 160, 170, 180, 190, 200, 210, 220, 230, 240, 250, 260, 270, 280, 290, 300 or more amino acid residues in length.

Moreover, other biologically-active portions of a protein, in which other regions of the protein are deleted, can be prepared by recombinant techniques and evaluated for one or more of the functional activities of a native protease.

In an embodiment, the protease has an amino acid sequence of one of the scaffolds described herein or one of the mutants of the scaffolds. The protease protein is substantially homologous to one of the scaffolds described herein or one of the mutants of the scaffolds, and retains the functional activity of the protein, yet differs in amino acid sequence due to natural allelic variation or mutagenesis. Accordingly, in another embodiment, the protease comprises an amino acid sequence at least about 45% homologous to the amino acid sequence of one of the scaffolds described herein or one of the mutants of the scaffolds, and retains the functional activity of one of the scaffolds described herein or one of the mutants of the scaffolds. In a preferred embodiment, the protease comprises an amino acid sequence at least about 90% homlogous to the amino acid sequence of one of the scaffolds. In another preferred embodiment, the protease comprises an amino acid sequence at least about 95% homlogous to the amino acid sequence of one of the scaffolds. In another preferred embodiment, the protease comprises an amino acid sequence at least about 99% homlogous to the amino acid sequence of one of the scaffolds.

### Determining Homology Between Two or More Sequences

To determine the percent homology of two amino acid sequences or of two nucleic acids, the sequences are aligned for optimal comparison purposes (*e.g*., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See,* Needleman and Wunsch, 1970. J Mol Biol 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g*., A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides protease chimeric or fusion proteins. As used herein, a protease "chimeric protein" or "fusion protein" comprises a protease polypeptide operatively-linked to a non-protease polypeptide. A " protease polypeptide" refers to a polypeptide having an amino acid sequence corresponding to one of the scaffolds described herein or one of the mutants of the scaffolds, whereas a "non-protease polypeptide" refers to a polypeptide having an amino acid sequence corresponding to a protein that is not substantially homologous to one of the scaffolds, *e.g*., a protein that is different from the scaffolds and that is derived from the same or a different organism. Within a protease fusion protein the protease polypeptide can correspond to all or a portion of a protease protein. In one embodiment, a protease fusion protein comprises at least one biologically-active portion of a protease protein. In another embodiment, a protease fusion protein comprises at least two biologically-active portions of a protease protein. In yet another embodiment, a protease fusion protein comprises at least three biologically-active portions of a protease protein. Within the fusion protein, the term "operatively-linked" is intended to indicate that the protease polypeptide and the non-protease polypeptide are fused in-frame with one another. The non-protease polypeptide can be fused to the N-terminus or C-terminus of the protease polypeptide.

In one embodiment, the fusion protein is a GST-protease fusion protein in which the protease sequences are fused to the N-terminus of the GST (glutathione S-transferase) sequences. Such fusion proteins can facilitate the purification of recombinant protease polypeptides.

In another embodiment, the fusion protein is a Fc fusion in which the protease sequences are fused to the N-terminus of the Fc domain from immunoglobulin G. Such fusion proteins can have increased pharmacodynamic properties *in vivo.*

In another embodiment, the fusion protein is a protease protein containing a heterologous signal sequence at its N-terminus. In certain host cells (*e.g*., mammalian host cells), expression and/or secretion of protease can be increased through use of a heterologous signal sequence.

A protease chimeric or fusion protein of the invention can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (*see, e.g.,* Ausubel et al. (eds.) CURRENT PROTOCOLS IN MOLECULAR BIOLOGY, John Wiley & Sons, 1992). Moreover, many expression vectors are commercially available that already encode a fusion moiety (*e.g*., a GST polypeptide). A protease-encoding nucleic acid can be cloned into such an expression vector such that the fusion moiety is linked in-frame to the protease protein.

### Protease Agonists and Antagonists

The invention also pertains to variants of the protease proteins that function as either protease agonists (*i.e*., mimetics) or as protease antagonists. Variants of the protease protein can be generated by mutagenesis (*e.g*., discrete point mutation or truncation of the protease protein). An agonist of the protease protein can retain substantially the same, or a subset of, the biological activities of the naturally occurring form of the protease protein. An antagonist of the protease protein can inhibit one or more of the activities of the naturally occurring form of the protease protein by, for example, cleaving the same target protein as the protease protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the protease proteins.

### Apoptosis

### Methods of Inhibiting Apoptosis

Also included in the invention are methods inhibiting apoptosis. Apoptosis, also known as programmed cell death, plays a role in development, aging and in various pathologic conditions. In developing organisms, both vertebrate and invertebrate, cells die in particular positions at particular times as part of the normal morphogenetic process. The process of apoptosis is characterized by, but not limited to, several events. Cells lose their cell junctions and microvilli, the cytoplasm condenses and nuclear chromatin marginates into a number of discrete masses. As the nucleus fragments, the cytoplasm contracts and mitochondria and ribosomes become densely compacted. After dilation of the endoplasmic reticulum and its fusion with the plasma membrane, the cell breaks up into several membrane-bound vesicles, apoptotic bodies, which are usually phagocytosed by adjacent bodies. As fragmentation of chromatin into oligonucleotides fragments is characteristic of the final stages of apoptosis, DNA cleavage patterns can be used as an *in vitro* assay for its occurrence (Cory, Nature 367: 317-18, 1994).

In one aspect, the invention provides a method of treating or preventing an apoptosis-associated disorder in a subject in need thereof by administering to the subject a therapeutically effective amount of a protease-inhibitor so apoptosis is inhibited. The subject can be *e.g*., any mammal, *e.g*., a human, a primate (*e.g*.human), mouse, rat, dog, cat, cow, horse, or pig. The term "therapeutically effective" means that the amount of protease-inhibitor, for example, which is used, is of sufficient quantity to ameliorate the apoptosis-associated disorder.

An apoptosis associated disorder includes, for example, immunodeficiency diseases, including AIDS/HIV, senescence, neurodegenerative diseases, any degenerative disorder, ischemic and reperfusion cell death, acute ischemic injury, infertility, wound-healing, and the like.

Many methods for measuring apoptosis, including those described herein, are known to the skilled artisan including, but not limited to, the classic methods of DNA ladder formation by gel electrophoresis and of morphologic examination by electron microscopy. The more recent and readily used method for measuring apoptosis is flow cytometry. Flow cytometry permits rapid and quantitative measurements on apoptotic cells. Many different flow cytometric methods for the assessment of apoptosis in cells have been described (Darzynkiewicz et al., Cytometry 13: 795-808, 1992). Most of these methods measure apoptotic changes in cells by staining with various DNA dyes (*i.e*. propidium iodide (PI), DAPI, Hoechst 33342), however, techniques using the terminal deoxynucleotidyl transferase (TUNNEL) or nick translation assays have also been developed (Gorczyca et al., Cancer Res 53: 1945-1951, 1993). Recently, rapid flow cytometric staining methods that use Annexin V for detection of phosphatidylserine exposure on the cell surface as a marker of apoptosis have become commercially available. The newest flow cytometric assays measure caspase-3 activity, an early marker of cells undergoing apoptosis and kits for performing this assays are commercially available (Nicholson et al., Nature 376: 37-43, 1995).

A protease can be administered to cleave a caspase thereby inhibiting its activity. In a preferred emboidment, the protease can be administered to cleave caspase-3. The protease can also cleave other proteins involved in apoptosis *e.g*., human cytochrome c, human Apaf-1, human caspase-9, human caspase-7, human caspase-6, human caspase-2, human BAD, human BID, human BAX, human PARP, or human p53. By cleaving these proteins, the protease thereby inactivates them. In this manner the protease can be used to inhibit apoptosis.

In another aspect apoptosis is inhibited in a cell by contacting a cell with a protease in an amount sufficient to inhibit apoptosis. The cell population that is exposed to, *i.e.,* contacted with, the protease can be any number of cells, *i.e*., one or more cells, and can be provided *in vitro, in vivo,* or *ex vivo.* The cells are contacted with the protease protein, or transfected with a polynucleotide that encodes the protease.

### Methods of Inducing Apoptosis

Also included in the invention are methods of inducing apoptosis. In one aspect apoptosis is induced in subject in need thereof by administering a protease in an amount sufficient to induce apoptosis. The subject can be *e.g.*, any mammal, a primate (*e.g.,* a human), mouse, rat, dog, cat, cow, horse, or pig. In various aspects the subject is susceptible to cancer or an autoimmune disorder.

A protease can be administered with an anti-angiogenic compound. Examples of an anti-angiogenic compound include, but are not limited to, a tyrosine kinase inhibitor, an epidermal-derived growth factor inhibitor, a fibroblast-derived growth factor inhibitor, a platelet-derived growth factor inhibitor, a matrix metalloprotease (MMP) inhibitor, an integrin blocker, interferon alpha, interferon-inducible protein 10, interleukin-12, pentosan polysulfate, a cyclooxygenase inhibitor, a nonsteroidal anti-inflammatory (NSAID), a cyclooxygenase-2 inhibitor, carboxyamidotriazole, tetrahydrocortizol, combretastatin A-4, squalamine, 6-O-chloroacetyl-carbonyl)-fumagillol, thalidomide, angiostatin, endostatin, troponin-1, an antibody to VEGF, platelet factor 4 or thrombospondin.

In some embodiments, the protease is further administered with a chemotherapeutic compound. Examples of chemotherapeutic compounds include, but are not limited to, paclitaxel, Taxol, lovastatin, minosine, tamoxifen, gemcitabine, 5-fluorouracil (5-FU), methotrexate (MTX), docetaxel, vincristin, vinblastin, nocodazole, teniposide, etoposide, adriamycin, epothilone, navelbine, camptothecin, daunonibicin, dactinomycin, mitoxantrone, amsacrine, epirubicin or idarubicin.

In another aspect, apoptosis is induced in a cell by contacting a cell with a protease in an amount sufficient to induce apoptosis. The cell population that is exposed to, *i.e.,* contacted with, the protease can be any number of cells, *i.e.,* one or more cells, and can be provided *in vitro, in vivo,* or *ex vivo.* The cells can be contacted with the protease protein, or transfected with a polynucleotide that encodes the protease.

Some disease conditions are related to the development of a defective down-regulation of apoptosis in the affected cells. For example, neoplasias result, at least in part, from an apoptosis-resistant state in which cell proliferation signals inappropriately exceed cell death signals. Furthermore, some DNA viruses such as Epstein-Barr virus, African swine fever virus and adenovirus, parasitize the host cellular machinery to drive their own replication. At the same time, they modulate apoptosis to repress cell death and allow the target cell to reproduce the virus. Moreover, certain disease conditions such as lymphoproliferative conditions, cancer including drug resistant cancer, arthritis, inflammation, autoimmune diseases and the like may result from a down regulation of cell death regulation. In such disease conditions, it is desirable to promote apoptotic mechanisms.

### EXAMPLES

### Example 1. Preparation and storage of I99A Granzyme B

The wild type rat granzyme B construct was prepared as described previously (Harris et al., JBC, 1998, (273):27364-27373). The following point mutations were introduced into the pPICZαA plasmid: N218A, N218T, N218V, I99A, I99F, I99R, Y174A, Y174V. Each mutation was confirmed by sequencing with primers to the 5'AOX and 3'AOX regions, followed by transformation into X33 cells and selection with Zeocin (Invitrogen, La Jolla CA). Expression and purification for each variant was identical to the previously described method for wild type rat granzyme B (Harris, et al., JBC, 1998, (273):27364-27373).

The protease rat granzyme B was mutated at Ile 99 to an Alanine using the QuikChange (Stratagene) method of site directed mutatgenesis. DNA primers to introduce the I99A mutation were: Forward primer: CCA GCG TAT AAT TCT AAG ACA GCC TCC AAT GAC ATC ATG CTG (SEQ ID NO:3) Reverse primer: CAG CAT GAT GTC ATT GGA GGC TGT CTT AGA ATT ATA CGC TGG (SEQ ID NO:5). A polymerase chain reaction was made containing the wild type double stranded DNA, the two primers overlapping the mutation, a reaction buffer, dNTP's and the DNA polymerase. After 30 rounds of annealing and amplification, the reaction was stopped. The enzyme DpnI was added to digest the wild type DNA containing a modified base pair, and the resulting nicked DNA strand is transformed into bacteria. A selection against Zeocin ensures only positive clones with grow. The mutation was confirmed by sequencing the granzyme B gene. The same protocol was used to make the remaining granzyme B mutants, with appropriate changes in the mutagenic primers.

The DNA containing the variant granzyme B proteases was transformed into Pichia pastoris X33 cells by the published protocol (Invitrogen) and the positive transformants were selected with Zeocin. The colony was transferred to a 1 L liquid culture and grown to a cell density of greater than OD600= 1.0. Protein expression was induced by the addition of 0.5% methanol and held constant over 72 hours. To purify the variant protease, the culture was centrifuged and the supernatant collected. Gravity based loading flowed the supernatant over a SP-Sepharose Fast Flow cation exchange column. The column was washed with 50 mM Mes, pH 6.0, 100 mM NaCl, and more stringently with 50 mM MES, pH 6.0, 250 mM NaCl. The protein was eluted with 50 mM MES, pH 6.0, 1 M NaCl and the column washed with 50 mM MES, pH 6.0, 2M NaCl and 0.5 M NaOH. The resulting protease was <90% pure. The final protease was exchanged and concentrated into 50mM MES, pH 6.0, 100 mM NaCl for storage at 4 °C.

Alternatively, following purification, each variant was quantitated by absorbance at 280nm (e280=13000 M-1cm-1), titrated with wildtype ecotin or M84D ecotin as previously described, exchanged into a buffer of 50 mM MES, pH 6.0 and 100 mM NaCl and stored at 4 °C.

### Example 2. Synthesis and Screening of ACC Positional Scanning Libraries

### ACC-Resin Synthesis

7-Fmoc-aminocoumarin-4-acetic acid was prepared by treating 7-aminocoumarin-4-acetic acid with Fmoc-Cl. 7-Aminocoumarin-4-acetic acid (10.0 g, 45.6 mmol) and H₂O (228 ml) were mixed. NaHCO₃ (3.92 g, 45.6 mmol) was added in small portions followed by the addition of acetone (228 ml). The solution was cooled with an ice bath, and Fmoc-Cl (10.7 g, 41.5 mmol) was added with stirring over the course of 1 h. The ice bath was removed and the solution was stirred overnight. The acetone was removed with rotary evaporation and the resulting gummy solid was collected by filtration and washed with several portions of hexane. ACC-resin was prepared by condensation of Rink Amide AM resin with 7-Fmoc-aminocoumarin-4-acetic acid. Rink Amide AM resin (21 g, 17 mmol) was solvated with DMF (200 ml). The mixture was agitated for 30 min and filtered with a filter cannula, whereupon 20% piperidine in DMF (200 ml) was added. After agitation for 25 min, the resin was filtered and washed with DMF (3 times, 200 ml each). 7-Fmoc-aminocoumarin-4-acetic acid (15 g, 34 mmol), HOBt (4.6 g, 34 mmol), and DMF (150 ml) were added, followed by diisopropylcarbodiimide (DICI) (5.3 ml, 34 mmol). The mixture was agitated overnight, filtered, washed (DMF, three times with 200 ml; tetrahydrofuran, three times with 200 ml; MeOH, three times with 200 ml), and dried over P₂O₅. The substitution level of the resin was 0.58 mmol/g (>95%) as determined by Fmoc analysis.

### P1-Diverse Library Synthesis

Individual P1-substituted Fmoc-amino acid ACC-resin (-25 mg, 0.013 mmol) was added to wells of a MultiChem 96-well reaction apparatus. The resin-containing wells were solvated with DMF (0.5 ml). After filtration, 20% piperidine in DMF solution (0.5 ml) was added, followed by agitation for 30 min. The wells of the reaction block were filtered and washed with DMF (three times with 0.5 ml). To introduce the randomized P2 position, an isokinetic mixture of Fmoc-amino acids [4.8 mmol, 10 eq per well; Fmoc-amino acid, mol %: Fmoc-Ala-OH, 3.4; Fmoc-Arg(Pbf)-OH, 6.5; Fmoc-Asn(Trt)-OH, 5.3; Fmoc-Asp(*O-t*-Bu)-OH, 3.5; Fmoc-Glu(*O-t*-Bu)-OH, 3.6; Fmoc-Gln(Trt)-OH, 5.3; Fmoc-Gly-OH, 2.9; Fmoc-His(Trt)-OH, 3.5; Fmoc-Ile-OH, 17.4; Fmoc-Leu-OH, 4.9; Fmoc-Lys(Boc)-OH, 6.2; Fmoc-Nle-OH, 3.8; Fmoc-Phe-OH, 2.5; Fmoc-Pro-OH, 4.3; Fmoc-Ser(*O-t*-Bu)-OH, 2.8; Fmoc-Thr(*O-t*-Bu)-OH, 4.8; Fmoc-Trp(Boc)-OH, 3.8; Fmoc-Tyr(*O-t*-Bu)-OH, 4.1; Fmoc-Val-OH, 11.3] was preactivated with DICI (390 µl, 2.5 mmol), and HOBt (340 mg, 2.5 mmol) in DMF (10 ml). The solution (0.5 ml) was added to each of the wells. The reaction block was agitated for 3 h, filtered, and washed with DMF (three times with 0.5 ml). The randomized P3 and P4 positions were incorporated in the same manner. The Fmoc of the P4 amino acid was removed and the resin was washed with DMF (three times with 0.5 ml) and treated with 0.5 ml of a capping solution of AcOH (150 µl, 2.5 mmol), HOBt (340 mg, 2.5 mmol), and DICI (390 µl, 2.5 mmol) in DMF (10 ml). After 4 h of agitation, the resin was washed with DMF (three times with 0.5 ml) and CH₂Cl₂ (three times with 0.5 ml), and treated with a solution of 95:2.5:2.5 TFA/TIS/H₂O. After incubation for 1 h the reaction block was opened and placed on a 96-deep-well titer plate and the wells were washed with additional cleavage solution (twice with 0.5 ml). The collection plate was concentrated, and the material in the substrate-containing wells was diluted with EtOH (0.5 ml) and concentrated twice. The contents of the individual wells were lyophilized from CH₃CN/H₂O mixtures. The total amount of substrate in each well was conservatively estimated to be 0.0063 mmol (50%) on the basis of yields of single substrates.

### P1-Fixed Library Synthesis

Multigram quantities of P1-substituted ACC-resin could be synthesized by the methods described. Fmoc-amino acid-substituted ACC resin was placed in 57 wells of a 96-well reaction block: sublibraries were denoted by the second fixed position (P4, P3, P2) of 19 amino acids (cysteine was omitted and norleucine was substituted for methionine). Synthesis, capping, and cleavage of the substrates were identical to those described in the previous section, with the exception that for P2, P3, and P4 sublibraries, individual amino acids (5 eq of Fmoc-amino acid monomer, 5 eq of DICI, and 5 eq of HOBt in DMF), rather than isokinetic mixtures, were incorporated in the spatially addressed P2, P3, or P4 positions.

Preparation of the complete diverse and P1-fixed combinatorial libraries was carried out as described above. The library was aliquoted into 96-well plates to a final concentration of 250 µM. Variant proteases were diluted in granzyme activity buffer (50 mM Na Hepes, pH 8.0, 100 mM NaCl, 0.01% Tween-20) to concentrations between 50 nM and 1 µM. Initial activity against Ac-IEPD-AMC was used to adjust the variant protease concentration to one approximately equal to 50 nM wild type rat granzyme B. Enzymatic activity in the P1-Asp library was assayed for one hour at 30 °C on a Spectra-Max Delta flourimeter (company name). Excitation and emission were measured at 380 nm and 460 nm, respectively.

### Example 3. Individual Kinetic Measurements of I99A Granzyme B

Individual kinetic measurements were performed using a Spectra-Max Delta fluorimeter. Each protease was diluted to between 50 nM and 1 µM in assay buffer. All ACC substrates were diluted with MeSO to between 5 and 500 µM, while AMC substrates were diluted to between 20 and 2000 µM. Each assay contained less than 5% MeSO. Enzymatic activity was monitored every 15 seconds at excitation and emission wavelengths of 380 nm and 460 nm, respectively, for a total of 10 minutes. All assays were performed in 1 % DMSO.

This method was used to screen I99A granzyme B. I99A granzyme was profiled in a positional scanning combinatorial substrate library to determine the effect of the mutation. The library was prepared as described above and aliquoted into 96-well plates to a final concentration of 250 µM. The variant protease was diluted in granzyme activity buffer (50 mM Na Hepes, pH 8.0, 100 mM NaCl, 0.01 % Tween-20) to concentrations between 50 nM and 1 µM. Initial activity against Ac-IEPD-AMC was used to adjust the variant protease concentration to one approximately equal to 50 nM wild type rat granzyme B. Enzymatic activity in the P1-Asp library was assayed for one hour at 30 °C on the Spectra-Max Delta fluorimeter. Excitation and emission were measured at 380 nm and 460 nm, respectively. The profiles of the granzyme B variants were compared to the wild type profile and the differences determined. For the I99A mutant, for example, the specificity at the P2 amino acid was markedly changed from the wild type. The former broad preference with a slight preference for proline is replaced with a strong preference for hydrophobic residues such as Phe and Tyr. The selectivity of the variant protease was also changed. The wild type was promiscuous at the P2 subsite, hydrolyzing substrates that contain any amino acid at that site. The I99A protease is much more selective. A Phe at the P2 site is preferred to a much higher degree than any other amino acid (*See* Table 5, above).

### Example 4. Proteolytic cleavage and inactivation of tumor necrosis factor and tumor necrosis factor receptor.

### Receptor cleavage.

Freshly isolated neutrophils (PMN) were resuspended at 1x10⁷ cells/ml in RPMI 1640 with 0.2% fetal calf serum (FCS) and incubated with various concentrations of protease, specific for the stalk region of TNF-R1 or TNF-R2. After a 1 to 40 min incubation at 37 °C, protease inhibitors were added to stop the reaction and the amount of TNF-R released into the media was quantitated using ELISA (Roche).

### TNF cleavage.

¹²⁵I-TNF (40,000 cpm) was incubated with varying concentrations of protease and then samples were boiled in SDS-PAGE sample buffer and examined on a 12% polyacrylamide gel. Gels were dried and exposed to x-ray film(Kodak) at -70 °C.

### TNF binding assay.

¹²⁵I-TNF or PMN were incubated with varying concentrations of proteases as above. The binding of ¹²⁵I-TNF exposed to proteases to normal PMN, or the binding of normal ¹²⁵I-TNF to PMN exposed to proteases, was quantitated using scintillation. Briefly, 10⁵ cells were incubated with varying concentrations of ¹²⁵I-TNF in 96-well filter plates (Millipore) in the presence of protease inhibitors. Cells were then washed three times by vacuum aspiration and then 30 µl of scintillation fluid (Wallac) was added to each well. Scintillation was then counted on a Wallac Microbeta scintillation counter. (Adapted from van Kessel et al., J. Immunol. (1991) 147: 3862-3868 and Porteau et al., JBC (1991) 266:18846-18853).

### Example 5. Selection of Enzymes Capable of Peptide Sequence Specific Target

### Cleavage Using Protease Phage Display

The phagemid is constructed such that it (i) carries all the genes necessary for M13 phage morphogenesis; (ii) it carries a packaging signal which interacts with the phage origin of replication to initiate production of single-stranded DNA; (iii) it carries a disrupted phage origin of replication; and (iv) it carries an ampicillin resistance gene.

The combination of an inefficient phage origin of replication and an intact plasmid origin of replication favors propagation of the vector in the host bacterium as a plasmid (as RF, replicating form, DNA) rather than as a phage. It can therefore be maintained without killing the host. Furthermore, possession of a plasmid origin means that it can replicate independent of the efficient phage-like propagation of the phagemid. By virtue of the ampicillin resistance gene, the vector can be amplified which in turn increases packaging of phagemid DNA into phage particles.

Fusion of the protease gene to either the gene 3 or gene 8 M13 coat proteins can be constructed using standard cloning methods. (Sidhu, Methods in Enzymology, 2000, V328, p333). A combinatorial library of variants within the gene encoding the protease is then displayed on the surface of M13 as a fusion to the p3 or p8 M13 coat proteins and panned against an immobilized, aldehyde-containing peptide corresponding to the target cleavage of interest. The aldehyde moiety will inhibit the ability of the protease to cleave the scissile bond of the protease, however this moiety does not interfere with protease recognition of the peptide. Variant protease-displayed phage with specficity for the immobilized target peptide will bind to target peptide coated plates, whereas non-specific phage will be washed away. Through consecutive rounds of panning, proteases with enhanced specificty towards the target sequence can be isolated. The target sequence can then be synthesized without the aldehyde and isolated phage can be tested for specific hydrolyis of the peptide.

### Example 6. The synthesis and fluorescence screening of libraries.

### A. P1-diverse Library

### A(i). Synthesis

Individual P1-substituted Fmoc-amino acid ACC-resin (ca. 25 mg, 0.013 mmol) was added to wells of a Multi-Chem 96-well reaction apparatus. The resin-containing wells were solvated with DMF (0.5 mL). A 20% piperidine in DMF solution (0.5 mL) was added followed by agitation for 30 min. The wells of the reaction block were filtered and washed with DMF (3x 0.5 mL). In order to introduce the randomized P2 position, an isokinetic mixture (Ostresh, J. M., et al., (1994) Biopolymers 34:1681-9) of Fmoc-amino acids (4.8 mmol, 10 equiv/well; Fmoc-amino acid, mol %: Fmoc-Ala-OH, 3.4; Fmoc-Arg(Pbf)-OH, 6.5; Fmoc-Asn(Trt)-OH, 5.3; Fmoc-Asp(O-t-Bu)-OH, 3.5; Fmoc-Glu(O-t-Bu)-OH, 3.6; Fmoc-Gln(Trt)-OH, 5.3; Fmoc-Gly-OH, 2.9; Fmoc-His(Trt)-OH, 3.5; Fmoc-Ile-OH, 17.4; Fmoc-Leu-OH, 4.9; Fmoc-Lys(Boc)-OH, 6.2; Fmoc-Nle-OH, 3.8; Fmoc-Phe-OH, 2.5; Fmoc-Pro-OH, 4.3; Fmoc-Ser(O-t-Bu)-OH, 2.8; Fmoc-Thr(O-t-Bu)-OH, 4.8; Fmoc-Trp(Boc)-OH, 3.8; Fmoc-Tyr(O-t-Bu)-OH, 4.1; Fmoc-Val-OH, 11.3) was pre-activated with DICI (390 µL, 2.5 mmol), and HOBt (340 mg, 2.5 mmol) in DMF (10 mL). The solution (0.5 mL) was added to each of the wells. The reaction block was agitated for 3 h, filtered, and washed with DMF (3.times.0.5 mL). The randomized P3 and P4 positions were incorporated in the same manner. The Fmoc of the P4 amino acid was removed and the resin was washed with DMF (3x 0.5 mL), and treated with 0.5 mL of a capping solution of AcOH (150 µL, 2.5 mmol), HOBt (340 mg, 2.5 mmol) and DICI (390 µL, 2.5 mmol) in DMF (10 mL). After 4 h of agitation, the resin was washed with DMF (3x 0.5 mL), CH₂Cl₂ (3x 0.5 mL), and treated with a solution of 95:2.5:2.5 TFA/TIS/H₂O. After incubating for 1 h the reaction block was opened and placed on a 96 deep-well titer plate and the wells were washed with additional cleavage solution (2x 0.5 mL). The collection plate was concentrated, and the substrate-containing wells were diluted with EtOH (0.5 mL) and concentrated twice. The contents of the individual wells were lyophilized from CH₃CN:H₂O mixtures. The total amount of substrate in each well was conservatively estimated to be 0.0063 mmol (50%) based upon yields of single substrates.

### A(ii). EnzymaticAssay of Library

The concentration of proteolytic enzymes was determined by absorbance measured at 280 nm (Gill, S. C., et al., (1989) Anal Biochem 182:319-26). The proportion of catalytically active thrombin, plasmin, trypsin, uPA, tPA, and chymotrypsin was quantitated by active-site titration with MUGB or MUTMAC (Jameson, G. W., et al., (1973) Biochemical Journal 131:107-117).

Substrates from the PS-SCLs were dissolved in DMSO. Approximately 1.0x10⁻⁹ mol of each P1-Lys, P1-Arg, or P1-Leu sub-library (361 compounds) was added to 57 wells of a 96-well microfluor plate (Dynex Technologies, Chantilly, Va.) for a final concentration of 0.1 µM. Approximately 1.0x10⁻¹⁰ mol of each P1-diverse sub-library (6859 compounds) was added to 20 wells of a 96-well plate for a final concentration of 0.01 µM in each compound. Hydrolysis reactions were initiated by the addition of enzyme (0.02 nM-100 nM) and monitored fluorometrically with a Perkin Elmer LS50B Luminescence Spectrometer, with excitation at 380 nm and emission at 450 nm or 460 nm. Assays of the serine proteases were performed at 25 °C. in a buffer containing 50 mM Tris, pH 8.0, 100 mM NaCl, 0.5 mM CaCl₂, 0.01% Tween-20, and 1% DMSO (from substrates). Assay of the cysteine proteases, papain and cruzain, was performed at 25 °C. in a buffer containing 100 mM sodium acetate, pH 5.5, 100 mM NaCl, 5 mM DTT, 1 mM EDTA, 0.01% Brij-35, and 1% DMSO (from substrates).

### B. Profiling Proteases with a P1-diverse Library of 137.180 Substrate Sequences

To test the possibility of attaching all amino acids to the P1-site in the substrate sequence a P1-diverse tetrapeptide library was created. The P1-diverse library consists of 20 wells in which only the P1-position is systematically held constant as all amino acids; excluding cysteine and including norleucine. The P2, P3, and P4 positions consist of an equimolar mixture of all amino acids for a total of 6,859 substrate sequences per well. Several serine and cysteine proteases were profiled to test the applicability of this library for the identification of the optimal P1 amino acid. Chymotrypsin showed the expected specificity for large hydrophobic amino acids. Trypsin and thrombin showed preference for P1-basic amino acids (Arg>Lys). Plasmin also showed a preference for basic amino acids (Lys>Arg). Granzyme B, the only known mammalian serine protease to have P1-Asp specificity, showed a distinct preference for aspartic acid over all other amino acids, including the other acidic amino acid, Glu. The P1-profile for human neutrophil elastase has the canonical preference for alanine and valine. The cysteine proteases, papain and cruzain showed the broad P1-substrate sequence specificity that is known for these enzymes, although there is a modest preference for arginine.

### Example 7. Screening for cleavage of individual substrates

Mutant proteases that match the desired specificity profiles, as determined by substrate libraries, are assayed using individual peptide substrates corresponding to the desired cleavage sequence. Individual kinetic measurements are performed using a Spectra-Max Delta fluorimeter (Molecular Devices). Each protease is diluted to between 50 nM and 1 µM in assay buffer. All ACC substrates are diluted with MeSO to between 5 and 500 µM, while AMC substrates are diluted to between 20 and 2000 µM. Each assay contain less than 5% MeSO. Enzymatic activity is monitored every 15 seconds at excitation and emission wavelengths of 380 nm and 460 nm, respectively, for a total of 10 minutes. All assays are performed in 1% DMSO.

### Example 8. Screening for cleavage of full-length proteins

Variant proteases are assayed to ascertain that they will cleave the desired sequence when presented in the context of the full-length protein, and the activity of the target protein is assayed to verify that its function has been destroyed by the cleavage event. The cleavage event is monitored by SDS-PAGE after incubating the purified full-length protein with the variant protease. The protein is visualized using standard Coomasie blue staining, by autoradiography using radio labeled protein, or by Western blot using the appropriate antibody. Alternatively, if the target protein is a cell surface receptor, cells expressing the target protein are exposed to the variant protease. The cleavage event is monitored by lysing the cells and then separating the proteins by SDS-PAGE, followed by visualization by Western blot. Alternatively, the soluble receptor released by proteolysis is quantified by ELISA.

The cleavage of the tumor necrosis factor receptors 1 and 2 (TNF-R1 and TNF-R2) were measured using these techniques. Freshly isolated neutrophils (PMN) were resuspended at 1x10⁷ cells/ml in RPMI 1640 with 0.2% fetal calf serum (FCS) and incubated with various concentrations of protease, specific for the stalk region of TNF-R1 or TNF-R2. After an incubation of 1 to 40 min at 37 °C, protease inhibitors were added to stop the reaction and the amount of TNF-R released into the media was quantified using ELISA (Roche).

Although the invention has been described with respect to specific methods of making and using enzymes capable of cleaving target polypeptide sequences, it will be apparent that various changes and modifications may be made without departing from the invention.

### Cleavage Of TNF.

¹²⁵I-TNF (40,000 cpm) was incubated with varying concentrations of protease, samples were boiled in SDS-PAGE sample buffer and examined on a 12% polyacrylamide gel. The gels were dried and exposed to x-ray film(Kodak) at-70 °C.

### TNF Binding Assay.

¹²⁵I-TNF or PMN were incubated with varying concentrations of proteases as above. The binding of ¹²⁵I-TNF exposed to proteases to normal PMN, or the binding of normal ¹²⁵I-TNF to PMN exposed to proteases, was quantified using scintillation. Briefly, 10⁵ cells were incubated with varying concentrations of ¹²⁵I-TNF in 96-well filter plates (Millipore) in the presence of protease inhibitors. Cells were washed three times by vacuum aspiration and 30 µL of scintillation fluid (Wallac) was added to each well. Scintillation was counted on a Wallac Microbeta scintillation counter. (Adapted from van Kessel et al., J. Immunol. (1991) 147: 3862-3868 and Porteau et al., JBC (1991) 266:18846-18853).

### Example 9. Identification of target proteins and cleavage sites therein

Proteins targeted for cleavage and inactivation are identified by the following criteria: 1) the protein is involved in pathology; 2) there is strong evidence the protein is the critical point of intervention for treating the pathology; 3) proteolytic cleavage of the protein will likely destroy its function. Cleavage sites within target proteins are identified by the following criteria: 1) they are located on the exposed surface of the protein; 2) they are located in regions that are devoid of secondary structure (*i.e.* β sheets or α helices), as determined by atomic structure or structure prediction algorithms; these regions tend to be loops on the surface of proteins or stalks on cell surface receptors; 3) they are located at sites that are likely to inactivate the protein, based on its known function. Cleavage sequences can be four residues in length to match the extended substrate specificity of many serine proteases, but can be longer or shorter.

### Tumor Necrosis Factor And Tumor Necrosis Factor Receptor

Tumor necrosis factor (TNF) is a pro-inflammatory cytokine that is primarily produced by monocytes, macrophages, and lymphocytes. TNF initiates signal transduction by interacting with either of two surface bound receptors, the p55 tumor necrosis factor receptor (TNF-R1) and the p75 tumor necrosis factor receptor (TNF-R2). TNF plays a central part in the pathophysiology of rheumatoid arthritis (RA), and is found at high concentrations of the synovium and synovial fluid of patients with RA. TNF signaling events result in the production of other pro-inflammatory cytokines (II-1, II-6, GM-CSF), induces the production of metalloproteinases such as collagenase and stromelysin, and increases the proliferation and activity of osteoclasts; all of these events lead to synovitis and tissue and bone destruction. Both types of TNF receptors are shed from the cell's surface as soluble forms that retain their ligand binding ability. These soluble TNFRs can neutralize TNF activity both *in vitro* and *in vivo,* and are believed to act as natural inhibitors to attenuate TNF signaling.

### VEGFR.

Vascular endothelial growth factor (VEGF) is an endothelial cell-specific mitogen normally produced during embryogenesis and adult life. VEGF is a significant mediator of angiogenesis in a variety of normal and pathological processes, including tumor development. Tumor vascularization is a vital process for the progression of a tumor to a stage from which it can metastasize. Three high affinity cognate receptors to VEGF have been identified: VEGFR-1/Flt-1, VEGFR-2/KDR, and VEGFR-3/Flt-4. VEGFRs are cell surface receptor tyrosine kinases that function as signaling molecules during vascular development.

### EGFR AND HER-2.

The ErbB family of receptor tyrosine kinases comprise four members: EGFR (Her-1), ErbB2 (Her-2), ErbB3 (Her-3) and ErbB4 (Her-4). All are essential for normal development and participate in the functioning of normal cells. ErbB receptors, particularly EGFR and ErbB2 are commonly deregulated in certain prevalent forms of human cancer. Dysregulation of ErbB signaling occurs by various mechanisms, including gene amplification, mutations that increase receptor transcription, or mutations that increase receptor activity. Activation of the ErbB receptors through the binding of the epidermal growth factor (EGF) results in downstream signaling through the mitogen-activated protein kinase (MAPK) and the Akt/phosphoinositide 3-kinase (PI3-kinase) pathways, ultimately leading to cell proliferation, differentiation, and angiogenesis.

### Potential Proteolytic Cleavage Sites.

Proteolytic cleavage sites for the proteins described above are shown below in Table 7.

**Table 7. Cleavage sequences for selected disease related proteins**

| **Target** | **Cleavage sequence** | **Region** | **Indication** |
|---|---|---|---|
| TNF-α | AEAK | loop | Rheumatoid arthritis, Crohn's disease, Inflammatory bowel disease, Psoriasis |
| TNF-R1 | ENVK | stalk | |
| | GTED | stalk | |
| TNF-R2 | SPTR | stalk | |
| | VSTR | stalk | |
| | STSF | stalk | |
| HER-2 | KFPD | stalk | Breast cancer |
| | AEQR | stalk | |
| EGFR | KYAD | stalk | Lung, breast, bladder, prostate, colorectal, kidney, head & neck cancer |
| | NGPK | stalk | |
| VEGFR-1 | SSAY | stalk | |
| | GTSD | stalk | |
| VEGFR-2 | AQEK | stalk | |
| | RIDY | loop | |

### Example 11. Cleavage Profile of Granzyme B I99A/N218A Mutant

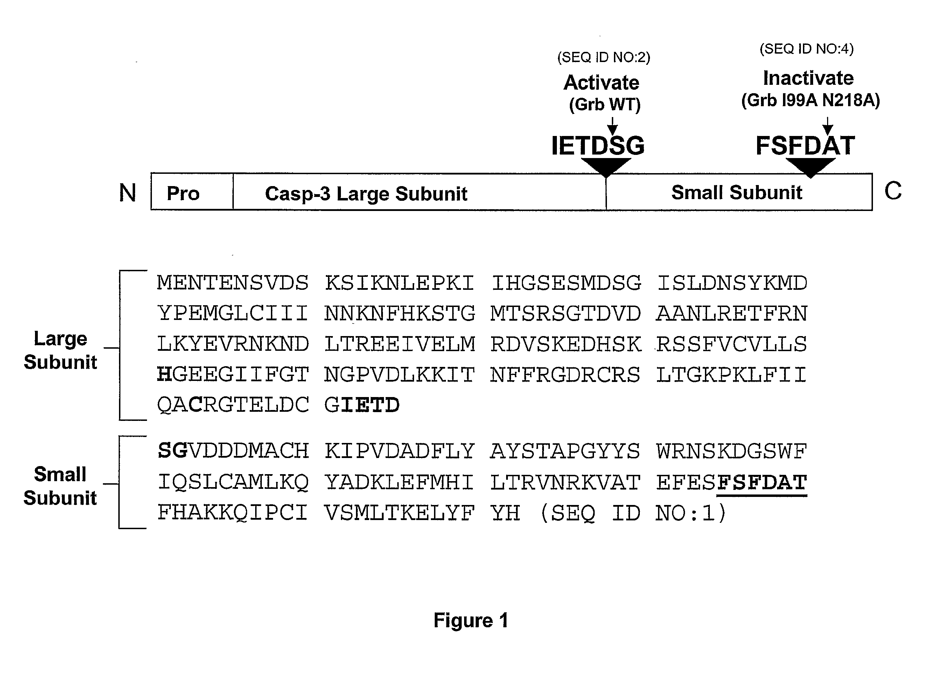

Figure 1 shows the sequence of caspase-3, a protein implicated in the apoptosis pathway of many cell types. Wild-type granzyme B cleaves caspase 3 between the aspartate and serine at residues 175 and 176 respectively. Mutations at positions 99 and 218 of granzyme B, change the specificity of this protease to cleavage between the aspartate and alanine of residues 263 and 264 respectively.

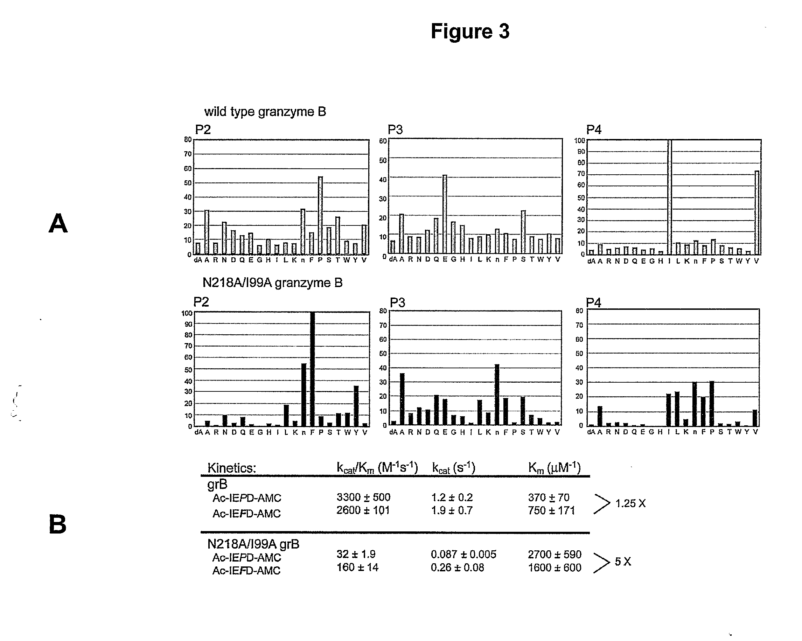

Figure 2 shows a crystallographic model of caspase-3 focusing on the inactivation sequence that is cleaved by I99A/N218A granzyme B at residues 260-265 (SEQ ID NO:2). The specificity of the mutated granzyme B is shown in Figure 3A with varying residues at the P2, P3 and P4 positions in a PS-SCL library. PS-SCL libraries in the form P4-P3-P2-Asp-AMC were assayed with wild-type and N218A/I99A granzyme B and the substrate specificity at each position is plotted by amino acid. The mutant showed (Figure 3B) a five times greater preference for phenylalanine at the P2 position over proline in the wild-type. Also, the mutant accommodates large hydrophobic amino acids including phenylalanine and leucine at the P4 position, where the wild-type generally accommodates only isoleucine and valine.

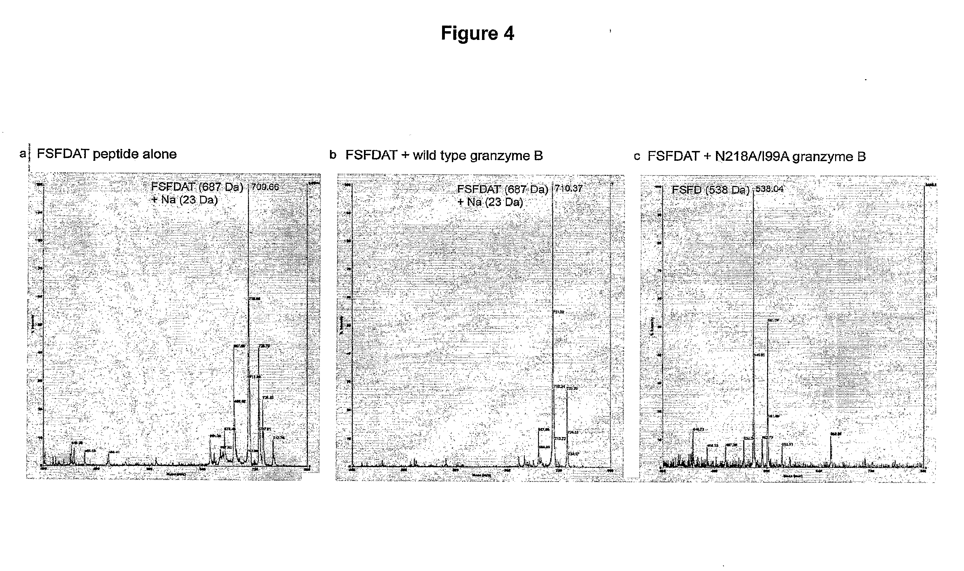

Figure 4 shows the cleavage of the NH₂-FSFDAT-COOH (SEQ ID NO:2) the caspase-3 inactivation sequence, made up of residues 260-265 of caspase-3, by MALDI mass spectrometry. The inactivation sequence was incubated with 100 nM wildtype granzyme B or 1 µM I99A/N218A for 18 hours. The first panel shows the molecular weight of the peptide alone. Shown is a peak representing the correct molecular weight for the uncleaved peptide. The second panel shows the results of the peptide being mixed with wild type granzyme B. The peak, again, represents the correct molecular weight for the uncleaved peptide, showing that wild-type granzyme B does not cleave the peptide. The third panel shows the results for the peptide with the N218A/I99A mutant granzyme B. Here, the peak has shifted to representing a cleavage product at 538.04, representing a cleavage product of the appropriate size (538 Da), for the cleaved peptide. The mutant granzyme B efficiently cleaves the peptide.

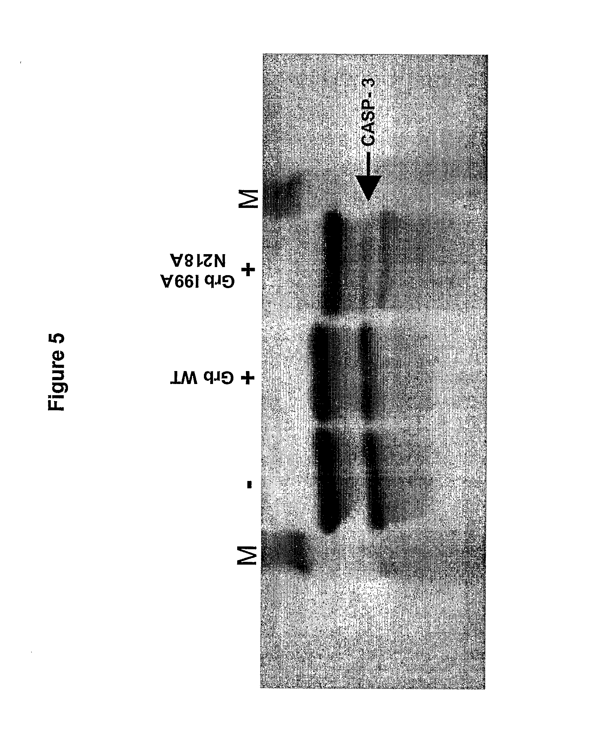

Figure 5 shows the results from three individual reactions run on an SDS-PAGE gel. Three individual tubes containing approximately 50 µM of caspase-3 were incubated in the presence of: in lane 1: buffer only; lane 2: wild-type granzyme B; lane 3: granzyme B I99A/N218A. Each reaction was terminated by the addition of 2X SDS sample buffer, then heated to 95 °C, and run on a tricine gel. The first lane shows caspase-3 alone. The second lane, shows caspase-3 with wild-type granzyme B. The third lane shows caspase-3 with the mutant granzyme B. The mutant is able to cleave the small subunit of caspase-3.

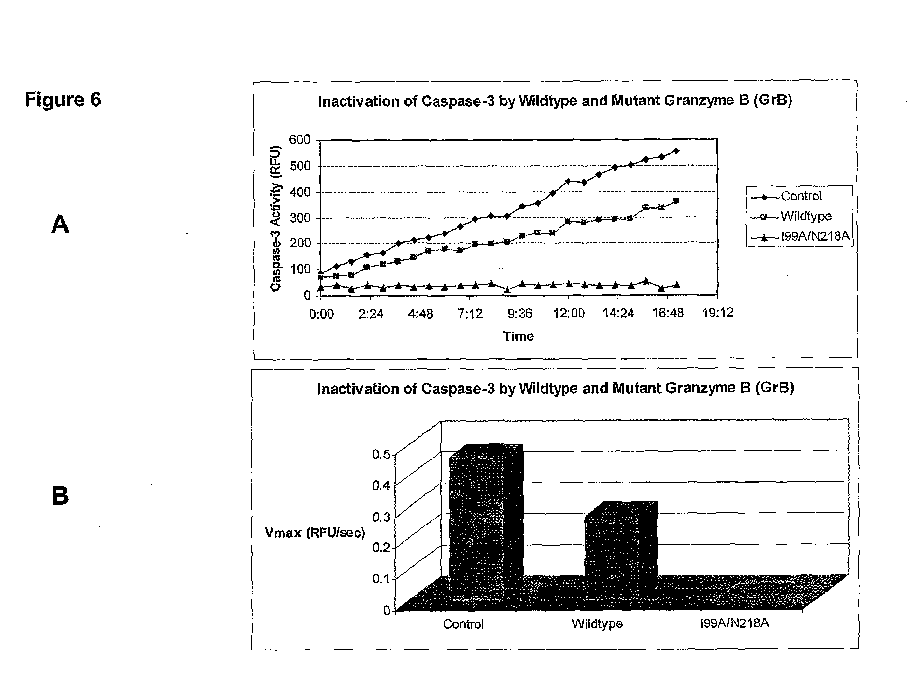

I99A/N218A granzyme B cleaved and inactivated full length caspase-3. Purified caspase-3 (2 µM) was incubated with no protease, 100 nM of wildtype granzyme B, or 1 µM I99A/N218A granzyme B for 18 hours in granzyme B activity buffer. 10 µL of each reaction was diluted in 90 µL of caspase-3 activity buffer and caspase-3 activity was assayed by cleavage of Ac-DEVD-AMC. Figure 6A shows a graph of caspase-3 activity plotted against time. I99A/N218A granzyme B inactivated caspase-3 to a very low level of activity. Wild-type granzyme B inactivated caspase-3 more than control, but did not have the effect that the mutant has on caspase-3 activity. This is also shown in Figure 6B, where Vmax of caspase-3 activity is shown derived from the data represented in Figure 6A. Vmax in the presence of the mutant granzyme B is approximately zero, wherein the wild-type only halves the Vmax relative to control.

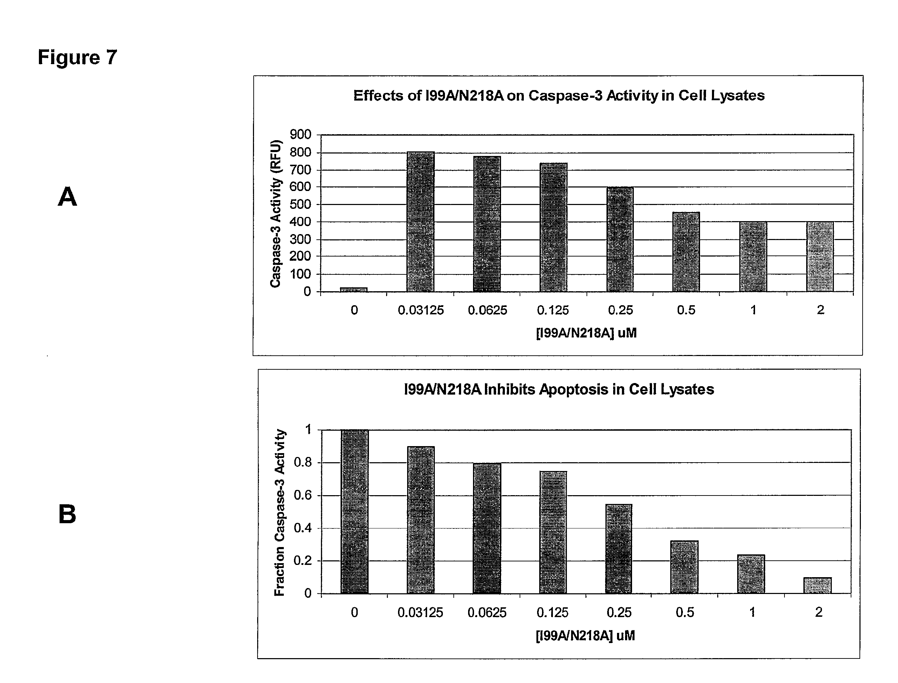

The mutant granzyme B was also effective in inhibiting caspase-3 activity and apoptosis in cell lysates containing caspase-3. In Figure 7A, indicated amounts of I99A/N218A granzyme B was added to cell lysates and incubated for 18 hours. Caspase-3 activity was then assayed by adding a fluorogenic substrate (Ac-DEVD-AMC) to a final concentration of 200 µM. At low concentrations the mutant activates caspase-3 by cleaving at the activation sequence (SEQ ID NO:4), but at high concentrations it inhibits caspase-3 by cleaving at the inactivation sequence. Thus, I99A/N218A granzyme B induces apoptosis at low concentrations but inhibits apoptosis at high concentrations. Figure 7A plots caspase-3 activity against increasing concentrations of I99A/N218A mutant granzyme B. As the concentration of the mutant granzyme B was increased in cell lysates, the caspase-3 activity decreased.

Apoptosis was induced in cell lysates by adding 100 nM of wildtype granzyme B, which activates caspase-3 by cleaving at the activation sequence, with or without the indicated amount of I99A/N218A granzyme B, and incubated for 18 hours. Caspase-3 activity was assayed by cleavage of Ac-DEVD-AMC. Data was normalized for the background caspase-3 activity induced by the I99A/N218A granzyme B. 100 nM of wild-type granzyme B was added to cell extracts in all samples, with or without increasing concentrations of I99A/N218A granzyme B as indicated in Figure 7B. As shown in Figure 7B, the mutant granzyme B antagonized the effect of wildtype granzyme B to induce apoptosis by inactivating caspase-3. Figure 7B shows a graph with the fraction of caspase-3 activity with varying concentrations of mutant granzyme B in the presence of 100 nM wild-type granzyme B. With increasing concentrations of mutant granzyme B, the caspase-3 activity decreased below the level it was in the presence of wild-type granzyme B alone.

### EQUIVALENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the spirit and scope of the invention as defined by the claims. The choice of screening method, protease scaffold, or library type is believed to be a matter of routine for a person of ordinary skill in the art with knowledge of the embodiments described herein. Other aspects, advantages, and modifications considered to be within the scope of the following claims.

Also described herein
(1) A method of identifying a protease which cleaves a substrate sequence the method comprising producing a library of protease sequences, each member of the library having a protease scaffold with N mutations relative to a wild-type scaffold sequence, measuring the activity of each member of the library in cleaving the substrate sequence, and comparing the activity of each member to the average activity of the library, thereby identifying which proteases have the highest cleavage activity, wherein N is a positive integer.
(2) The method of (1), wherein the protease is a serine or cysteine protease.
(3) The method of (1), wherein N is 1.
(4) The method of (1), wherein N is 1-5.
(5) The method of (1), wherein N is 5-10.
(6) The method of (1), wherein N is 10-20.
(7) The method of (1), wherein the protease scaffold has the amino acid sequence of one of the members of the group consisting of trypsin, chymotrypsin, substilisin, thrombin, plasmin, Factor Xa, uPA, tPA, MTSP-1, granzyme A, granzyme B, granzyme M, elastase, chymase, papain, neutrophil elastase, plasma kallikrein, urokinase type plasminogen activator, complement factor serine proteases, ADAMTS 13, neural endopeptidase/neprilysin, furin, and cruzain.
(8) The method of (1), wherein the target protein is involved with a pathology.
(9) The method of (8), wherein the pathology is a member of the group consisting of rheumatoid arthritis, sepsis, cancer, acquired immunodeficiency syndrome, respiratory tract infections, influenza, cardiovascular disease, and asthma.
(10) The method of (8), wherein the target protein is involved in a way that it causes the pathology.
(11) The method of (1), wherein the target protein is involved in apoptosis.
(12) The method of (11), wherein the target protein is caspase-3.
(13) The method of (1), wherein the activity of the detected protease is increased by at least 10-fold compared to the average activity of the library.
(14) The method of (1), wherein the activity of the detected protease is increased by at least 100-fold compared to the average activity of the library.
(15) The method of (1), wherein the activity of the detected protease is increased by at least 1000-fold compared to the average activity of the library.
(16) The method of (1), further comprising the steps of: providing two or more members of the protease library identified with increased cleavage activity, combining the mutations on a first scaffold with the mutations on a second scaffold to produce a third scaffold; and identifying whether the combination produces a combined specificity protease that has increased cleavage activity in regards to the substrate sequence.
(17) A method of identifying a substrate sequence which is specifically cleaved by a protease, the method comprising producing a library of substrate sequences, each member of the library having randomized amino acid sequences, measuring the degree to which each member of the library is cleaved by the protease, thereby detecting which substrate sequences are cleaved most efficiently by the protease compared to the average cleavage of other members of the library.
(18) The method of (17), wherein the protease is a serine protease.
(19) The method of (17), wherein each member of the substrate sequence library is 4 amino acids long.
(20) The method of (17), wherein each member of the substrate sequence library is 5-10 amino acids long.
(21) The method of (17), wherein each member of the substrate sequence library is 15 amino acids long.
(22) The method of (17), wherein each member of the substrate sequence library is 20 amino acids long.
(23) The method of (17), wherein a target protein comprises the substrate sequence.
(24) The method of (23), wherein the target protein is involved in a pathology.
(25) The method of (24), wherein the pathology is a member of the group consisting of rheumatoid arthritis, sepsis, cancer, acquired immunodeficiency syndrome, respiratory tract infections, influenza, cardiovascular disease, inflammation, and asthma.
(26) The method of (24), wherein the target protein is involved in such a way that it causes the pathology.
(27) The method of (17), wherein the efficiency of cleavage of the detected substrate sequence is increased by at least 10-fold compared to the average activity of the library.
(28) The method of (17), wherein the efficiency of cleavage of the detected substrate sequence is increased by at least 100-fold compared to the average activity of the library.
(29) The method of (17), wherein the efficiency of cleavage of the detected substrate sequence is increased by at least 1000-fold compared to the average activity of the library.
(30) A method for treating a patient with a pathology, the method comprising administering to the patient a wild-type protease with N mutations, wherein the protease is administered in an amount sufficient to cleave a target protein that is involved with the pathology, wherein cleavage of the protein causes treatment of the pathology, and wherein N is a positive integer.
(31) The method of (30), wherein N is 1.
(32) The method of (30), wherein N is 1-5.
(33) The method of (30), wherein N is 5-10.
(34) The method of (30), wherein N is 10-20.
(35) The method of (30), wherein the pathology is a member of the group consisting of rheumatoid arthritis, sepsis, cancer, acquired immunodeficiency syndrome, respiratory tract infections, influenza, cardiovascular disease, inflammation, and asthma.
(36) The method of (30), wherein the protease is a serine protease.
(37) The method of (30), wherein the target protein causes the pathology.
(38) The method of (30), wherein the patient is a mammal.
(39) The method of (30), wherein the patient is a human.
(40) The method of (30), wherein the target protein in a member of the group consisting of tumor necrosis factor receptor, interleukin-1, interleukin-1 receptor, interleukin-2, interleukin-2 receptor, interleukin-4, interleukin-4 receptor, interleukin-5, interleukin- 5 receptor, interleukin- 12, interleukin- 12 receptor, interleukin- 13, interleukin- 13 receptor, p-selectin, p-selectin glycoprotein ligand, Substance P, the Bradykinins, factor IX, immunoglobulin E, immunoglobulin E receptor, CCR5, CXCR4, glycoprotein 120, glycoprotein 41, CD4, hemaglutinin, respiratory syncytium virus fusion protein, B7, CD28, CD2, CD3, CD4, CD40, vascular endothelial growth factor, VEGF receptor, fibroblast growth factor, FGF receptor, endothelial growth factor, EGF receptor, transforming growth factor, TGF receptor, CCR1, CXCR3, CCR2, Src, Akt, Bcl-2, BCR-Abl, glucagon synthase kinase-3, cdk-2, and cdk-4.
(41) A composition comprising a polypeptide 95% identical to the amino acid sequence of granzyme B, wherein the polypeptide has a mutation at at least one of positions 171, 174, 180, 215, 192, 218, 99, 57, 189, 190, or 226.
(42) The composition of (41), wherein said mutation is isoleucine replaced with alanine at position 99.
(43) The composition of (41), wherein said mutation is asparagine replaced with alanine at position 218.
(44) The composition of (41), wherein said mutations are isoleucine replaced with alanine at position 99 and asparagine replaced with alanine at position 218.

## Claims

1. A modified protease, or a biologically active portion thereof, comprising a mutation that alters substrate specificity for a target protein, wherein:
the modified protease comprises a mutation in a serine protease scaffold at an amino acid position that is a structural determinant of specificity (S1-S4) selected from among amino acid positions between 58 and 64, 97, 98, 99, 100, 171, 174, 180, 189, 190, 191, 215, 217, 218 and 226, by chymotrypsin numbering;
the protease scaffold is selected from among acrosin; anionic trypsin (II); brain serine proteinase 2; cathepsin G; cathepsin G (CTSG); Cationic trypsin (PRSS1); chymase; chymase; chymopasin; chymotrypsin; chymotrypsin B; coagulation factor IXa; coagulation factor VIIa; coagulation factor Xa; coagulation factor XIa; coagulation factor XIIa; complement component 2 (C2); complement component activated C1r; complement component activated C1s; complement factor I; complement factor B; complement factor D; complement factor serine proteases; Corin; DESC1 protease; elastase; enteropeptidase chymotrypsin C; epitheliasin (TMPRSS2); furin; granzyme A; granzyme A; granzyme H; granzyme K; granzyme M; granzyme M; hepsin;hepatocyte growth factor activator; human-type testisin (PRSS21); hyaluronan-binding serine protease; kallikrein (hK10); kallikrein 1; kallikrein 11; kallikrein 12 (KLK12); kallikrein 3; kallikrein hK13; kallikrein hK14 (KLK14); kallikrein hK15; kallikrein hK2; kallikrein hK5 (KLK5); kallikrein hK9; LCLP proteinase; LOC144757 peptidase; mannan-binding lectin-associated serine protease 1 (MASP1): ; mannan-binding lectin-associated serine protease 2; mannan-binding lectin-associated Serine protease 3; Marapsin; matriptase; membrane type serine protease-1 (MTSP-1); membrane-type mosaic serine protease; Mername-AA031 peptidase; mername-AA035 peptidase; Mername-AA038 Peptidase; mername-AA122 peptidase.; Mername-AA128 Peptidase; mername-AA169 peptidase; mername-AA 171 peptidase; mername-AA174 peptidase; mername-AA175 petidase; mername-AA178 peptidase; mername-AA180 peptidase; mername-AA182 peptidase ; mesotrypsin; Myeloblastin PRTN3; neuropsin; neurosin; neurotrypsin; neutrophil elastase; pancreatic elastase; pancreatic elastase II form B; pancreatic endopeptidase E; pancreatic endopeptidase E form B (B); plasma kallikrein; plasmin; prostase; prostasin; protein C ; respiratory; spinesin; stratum corneum chymotryptic enzyme; subtilisin; thrombin; tissue plasminogen activator (tPA); Transmembrane protease serine 3 (TMPRSS3); Transmembrane protease Serine 4 (TMPRSS4); trypsin; trypsin-like enzyme; tryptase alpha; tryptase beta 1 (TPSB 1); tryptase beta 2 (2); tryptase beta 2 (I); Tryptase chromosome 21; tryptase delta (TPSD1); tryptase gamma 1 (TPSG1); Tryptase homologue 2; Tryptase homologue 3; umbilical vein proteinase and urokinase plasminogen activator (uPA); and the modified protease retains proteolytic activity.

2. The modified protease of claim 2, wherein the protease scaffold is urokinase plasminogen activator (uPA), MT-SP1 or chymase.

3. The modified protease of claim 1 or claim 2, wherein the cleavage activity, specificity, or the cleavage activity and specificity for a substrate sequence in the target protein is increased compared to the protease scaffold.

4. The modified protease of claim 3, wherein cleavage activity or specificity is increased by at least 10-fold, 100-fold or 1000-fold compared to the scaffold protease.

5. The modified protease of any of claims 1-4, wherein the target protein is selected from among a cytokine, receptor, fusion protein, kinase, and G protein-coupled receptor (GPCR).

6. The modified protease of any of claims 1-5, wherein the target protein is selected from among tumor necrosis factor receptor, interleukin-1, interleukin-1 receptor, interleukin-2, interleukin-2 receptor, interleukin-4, interleukin-4 receptor, interleukin-5, interleukin-5 receptor, interleukin-12, interleukin-12 receptor, interleukin-13, interleukin-13 receptor, p-selectin, p-selectin glycoprotein ligand, Substance P, the Bradykinins, factor IX, immunoglobulin E, immunoglobulin E receptor, CCR5, CXCR4, glycoprotein 120, glycoprotein 41, CD4, hemaglutinin, respiratory syncytium virus fusion protein, B7, CD28, CD2, CD3, CD4, CD40, vascular endothelial growth factor, VEGF receptor, fibroblast growth factor, FGF receptor, endothelial growth factor, EGF receptor, transforming growth factor, TGF receptor, CCR1, CXCR3, CCR2, Src, Akt, Bcl-2, BCR-Abl, glucagon synthase kinase-3, cytochrome c, Apaf-1, caspase-3, caspase-9, caspase-7, caspase-6, caspase-2, BAD, BID, BAX, PARP, p53, cdk-2, and cdk-4.

7. The modified protease of any of claims 1-6, that is a fusion or chimeric protein.

8. The modified protease of any of claims 1-7, that contains 1, 2, 3, 4, 5, 6, 7, 8, 9, 10, 11, 12, 13, 14, 15, 16, 17, 18, 19 or 20 mutations compared to the protease scaffold.

9. The modified protease of any of claims 1-8, wherein the protease scaffold is a biologically-active portion of a protease.

10. The modified protease of any of claims 1-9, wherein the protease comprises an amino acid sequence that is at least 90% homologous to the amino acid sequence of the wildtype protease scaffold.

11. The modified protease of any of claims 1-9, wherein the protease comprises an amino acid sequence that is at least 95% homologous to the amino acid sequence of the wildtype protease scaffold.

12. The modified protease of any of claims 1-9, wherein the protease comprises an amino acid sequence that is at least 99% homologous to the amino acid sequence of the wildtype protease scaffold.

13. A library of modified proteases of any of claims 1-12.

14. A pharmaceutical composition for treating a disease or pathology in a mammal, comprising a modified protease of any of claims 1-13, wherein:
the modified protease cleaves a target protein involved with the disease or pathology;
cleavage of the target protein treats the disease or pathology; and
the disease or pathology is selected from among rheumatoid arthritis, sepsis, cancer, acquired immunodeficiency syndrome, respiratory tract infection, influenza, cardiovascular disease, inflammation, asthma, neurodegenerative disease, ischemic and reperfusion cell death, degenerative disorders, and acute ischemic injury.

15. The pharmaceutical composition of claim 14, wherein the target protein involved in the disease or pathology is selected from among tumor necrosis factor receptor, interleukin-1, interleukin-1 receptor, interleukin-2, interleukin-2 receptor, interleukin-4, interleukin-4 receptor, interleukin-5, interleukin-5 receptor, interleukin-12, interleukin-12 receptor, interleukin-13, interleukin-13 receptor, p-selectin, p-selectin glycoprotein ligand, Substance P, the Bradykinins, factor IX, immunoglobulin E, immunoglobulin E receptor, CCR5, CXCR4, glycoprotein 120, glycoprotein 41, CD4, hemaglutinin, respiratory syncytium virus fusion protein, B7, CD28, CD2, CD3, CD4, CD40, vascular endothelial growth factor, VEGF receptor, fibroblast growth factor, FGF receptor, endothelial growth factor, EGF receptor, transforming growth factor, TGF receptor, CCR1, CXCR3, CCR2, Src, Akt, Bcl-2, BCR-Abl, glucagon synthase kinase-3, cytochrome c, Apaf-1, caspase-3, caspase-9, caspase-7, caspase-6, caspase-2, BAD, BID, BAX, PARP, p53, cdk-2, and cdk-4.
